(19) Europäisches Patentamt
European Patent Office
Office européen des brevets

(11) **EP 4 382 101 A1**

(12) **EUROPEAN PATENT APPLICATION**
published in accordance with Art. 153(4) EPC

(43) Date of publication:
**12.06.2024 Bulletin 2024/24**

(21) Application number: **22851498.0**

(22) Date of filing: **02.08.2022**

(51) International Patent Classification (IPC):
***A61K 31/175*** *(2006.01)* ***A61K 31/505*** *(2006.01)*
***A61K 31/44*** *(2006.01)* ***A61K 31/34*** *(2006.01)*
***A61K 31/381*** *(2006.01)* ***A61K 31/42*** *(2006.01)*
***A61K 31/4245*** *(2006.01)* ***A61K 31/425*** *(2006.01)*
***A61K 31/433*** *(2006.01)* ***A61P 25/02*** *(2006.01)*
***A61P 29/00*** *(2006.01)*

(52) Cooperative Patent Classification (CPC):
**A61K 31/175; A61K 31/34; A61K 31/381; A61K 31/42; A61K 31/4245; A61K 31/425; A61K 31/433; A61K 31/44; A61K 31/505; A61P 25/02; A61P 29/00**

(86) International application number:
**PCT/BR2022/050304**

(87) International publication number:
**WO 2023/010192 (09.02.2023 Gazette 2023/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.08.2021 US 202163228516 P**

(71) Applicants:
- **Eurofarma Laboratórios S.A.**
  **06696-000 Itapevi - SP (BR)**
- **Universidade Federal Do Rio De Janeiro - UFRJ**
  **21941-901 Rio de Janeiro (BR)**

(72) Inventors:
- **BARREIRO, Gabriela**
  **06696-000 São Paulo - SP (BR)**
- **SANT'ANA, Danilo Pereira De**
  **06709-320 São Paulo - SP (BR)**
- **GAMBA, Luis Eduardo Reina**
  **06696-000 São Paulo - SP (BR)**
- **FRAGA, Carlos Alberto Manssour**
  **21920-190 Rio de Janeiro - RJ (BR)**
- **BARREIRO, Eliezer Jesus De Lacerda**
  **21931-220 Rio de Janeiro - RJ (BR)**
- **LIMA, Lídia Moreira**
  **21931-220 Rio de Janeiro - RJ (BR)**

(74) Representative: **Engelhard, Markus**
**Boehmert & Boehmert**
**Anwaltspartnerschaft mbB**
**Pettenkoferstrasse 22**
**80336 München (DE)**

# (54) N-ACYLHYDRAZONE COMPOUNDS CAPABLE OF INHIBITING NAV1.7 AND/OR NAV1.8, PROCESSES FOR THE PREPARATION THEREOF, COMPOSITIONS, USES, METHODS FOR TREATMENT USING SAME, AND KITS

(57) The present invention relates to *N*-Acylhydrazone compounds that are Nav 1.7 and/or Nav 1.8 inhibitors. More specifically, the present invention relates to N-acylhydrazone compounds of Formula (I), wherein substituents R1 to R4 are independently selected from the groups defined in the specification, as well as to the processes for the preparation thereof, compositions comprising at least one of these compounds, uses, treatment methods for treating or preventing pain-related pathologies, and kits. The present invention lies in the fields of medicinal chemistry, organic synthesis, as well as in the treatment of pain-related disorders.

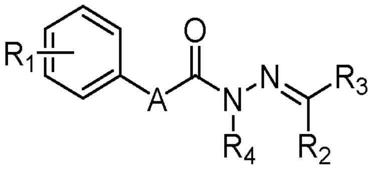 Formula (I)

## Description

## Field of invention

The present invention relates to *N*-acylhydrazone compounds that are Nav 1.7 and/or Nav 1.8 inhibitors, the processes for the preparation thereof, compositions containing these, uses, kits and treatment methods for treating or preventing pain-related pathologies. The present invention is applicable in the fields of medicinal chemistry, organic synthesis, as well as in the treatment of pain-related disorders.

## Background of Invention

Physiological pain is an important protective mechanism developed to alert the body to actual or potential injuries that may endanger its integrity. In general terms, physiological pain can be classified into nociceptive pain and inflammatory pain. Nociceptive pain is characterised by presenting a high activation threshold, which remains until the stimulus that generated it is eliminated. Inflammatory pain, which arises as a response to tissue damage, is characterised by a low activation threshold and is a consequence of the activity of cellular and molecular mediators of the inflammatory process in sensitizing nociceptors (Schaible. Langenbecks Arch. Surg. 2004, 389, 237). When these nociceptive processes remain in the absence of harmful stimuli or in response to non-harmful stimuli, the protective and repair role of pain loses its functionality, constituting a maladaptive framework of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or diseases of the central nervous system such as multiple sclerosis or Parkinson's disease (Ducreux. Brain, 2006, 129, 963). Peripheral neuropathic pain, on the other hand, can be caused by trauma, metabolic disorders, chemical neurotoxicity, infection or tumor invasion, among others. Among the most common neuropathic pain syndromes are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia and postsurgical neuralgia (Pak. Curr. Pain Headache Rep., 2018, 22(2), 9).

Currently, there is no specific treatment for the control of pathologies related to neuropathic pain. However, the first-line alternative consists of the use of opioid analgesics, and - as adjuvants - local anesthetics, anticonvulsants and antidepressants. Nevertheless, adverse effects and poor efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization allowing the generation and propagation of action potentials in neurons (and other electrically excitable cells) by controlling the flow of sodium ions across membranes. Structurally, voltage-gated sodium channels are heteromeric transmembrane proteins consisting of an $\alpha$ subunit and two helper $\beta$ subunits. The $\alpha$ subunit is organized into four homologous domains (I-IV) each with six transmembrane segments (S1-S6). The S4 segment of each domain is characterised by a conserved region of arginine residues, which act as sensors of the intra- and extracellular electrical environment of the neuron. This mechanism allows transforming alterations of the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine $\alpha$ subunits (Nav 1.1 - Nav 1.9) and four $\beta$ helper subunits ($\beta$1-$\beta$4) have been identified. The $\alpha$ subunits can also be classified as to their susceptibility to tetrodotoxin blockade (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these $\alpha$ subunits exhibit a differentiated expression and function profile such that some of them are essential for the proper functioning of organs such as the heart and/or brain. Thus, the non-selective blockage of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

In general terms, sodium channels are distributed mainly in the central and peripheral nervous system, in neurons and glia. Nav channels 1.1, 1.2 and 1.3 are mainly expressed in the brain. Nav 1.4 and Nav 1.5 channels are mainly found in skeletal and cardiac muscles, respectively. Nav 1.6 channels are expressed in the central and peripheral

nervous system, while Nav 1.9 channels are selectively expressed in C-type nociceptive fibers in the dorsal root ganglion. On the other hand, Nav 1.7 and Nav 1.8 channels are mainly found in the peripheral nervous system and are directly related to pain transmission processes (Law. Drug Discovery Today, 2019, 24(7), 1389; Bagal. Channels (Austin), 2015, 9(6), 360; Lera-Ruiz. J. Med. Chem., 2015, 58(18), 7093).

Nav 1.7 sodium channels are expressed broadly in the olfactory epithelium, sympathetic ganglion, and dorsal root ganglion, predominantly in the C and Aδ nociceptivefibers. A large amount of evidence supports the important role of Nav 1.7 sodium channels in pain transmission processes. For example, gain-of-function-related mutations in the gene (SCN9A), which encodes the Nav 1.7 sodium channel, are associated with extreme pain disorders such as congenital pain hypersensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels show increased expression in the peripheral nervous system, largely (but not exclusively) in C-type nociceptive fibers in the dorsal root ganglion. Recent evidence that includes elevated Nav 1.8 expression levels in chronic pain states, Nav 1.8 *knockout* animal data, and analgesic activity of desensitizing oligodeoxynucleotides specific for Nav 1.8, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the Nav 1.8 sodium channel in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

In this way, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels. However, they present a great structural diversity, a fact that does not allow a common pharmacophoric group to be established.

The patent literature contains several examples of compounds that act as sodium channel blockers. In particular, blockers of the selective sodium channels Nav 1.7 are described in US10550080, US9765029 and US10000475. Additionally, some documents describe selective blockers of Nav 1.8 sodium channels such as WO2020261114, WO2020092667, US9163042, WO2014120808, WO2014120815, WO2018213426, WO2019014352, WO2015006280 and US7928107. These documents disclose compounds having distinct structures of the present invention.

Still, there are patent documents describing dual inhibitors of Nav 1.7 and 1.8, including WO2018235851, US8629149, JP2017001991 that disclose, respectively, pyridyl amines, oxopiperazine derivatives and benzoxazolones. However, all of these prior art documents disclose compounds with structures and physicochemical characteristics distinct from the present invention.

In this context, it is advantageous to develop new alternatives of compounds that can act as inhibitors of Nav 1.7 and/or Nav 1.8 that have adequate pharmacological action and, preferably, provide mitigated adverse effects. Therefore, the present invention relates to N-acylhydrazone derivatives endowed with novel and inventive step as an alternative and/or complement to the treatment of pain-related diseases.

## Summary of Invention

The present invention discloses *N*-acylhydrazone compounds with inhibitory activity of Nav 1.7 and/or 1.8, against pain-related pathologies, in addition to compositions, uses, kits, treatment methods and related preparation processes.

The present invention relates to compound(s) of Formula (I):

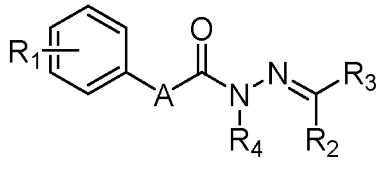

Formula (I)

or a pharmaceutically acceptable salt, hydrate, solvate, and isomer thereof, wherein:
A is selected from the group consisting of

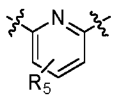 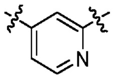 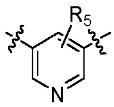 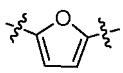 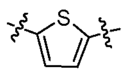 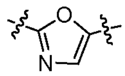 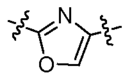

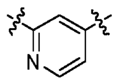 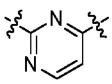 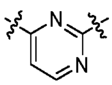 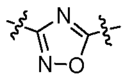 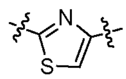 ou 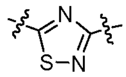

- R1 is selected from the group consisting of hydrogen, halogen, linear or branchedC1-6 alkoxy, 2-morpholinoethoxy, C3-6 cycloalkyloxy, or C1-5 haloalkyloxy;
- R2 and R4 are independently selected from the group consisting of hydrogen or branched or linear C1-6 alkyl;
- R3 is selected from the group consisting of hydrogen, heterocycle or substituted heterocycle, or R6;
- R5 is selected from the group consisting of hydrogen, halogen, trifluoromethyl.
- R6 is

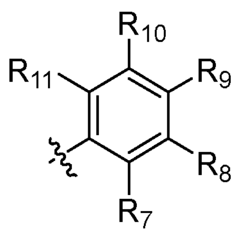

- R7, R8, R9, R10 andR11 are independently selected from the group consisting of hydrogen, halogen, linear or branched C1-6 alkoxy.

Additionally, the present invention also relates to compositions comprising one or more compound(s) of Formula (I) or pharmaceutically acceptable salts, solvates, and isomers thereof; and one or more pharmaceutically acceptable excipients.

Additionally, kits according to the present invention may comprise such compositions and application devices, which may include ampoules, syringes, and others. Alternatively, kits according to the present invention comprise more than one compound of Formula (I) arranged in one or more dosage forms, including without limitation, tablets, accompanied by instructions for administration.

The present invention further relates to methods of treating, preventing, alleviating, suppressing and/or controlling diseases related to neuropathic pain. Also taught are uses of compound(s) of general Formula (I) for preparing a medicament for the treatment of neuropathic pain-related pathologies. Finally, the present invention teaches processes of obtaining compound(s) of general Formula (I).

## Detailed description of invention

The present invention features in a first embodiment, the compound of Formula (I):

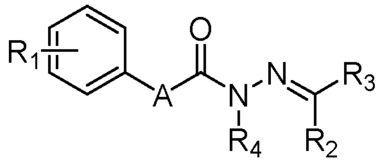

Formula (I)

or a pharmaceutically acceptable salt, hydrate, solvate, and isomer thereof, wherein:
A is selected from the group consisting of

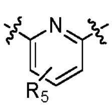 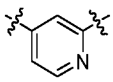 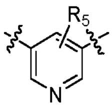 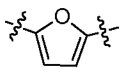 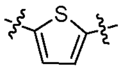 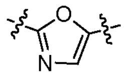 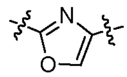

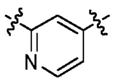 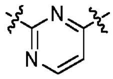 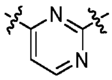 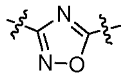 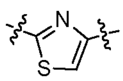 ou 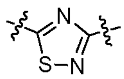

- R1 is selected from the group consisting of hydrogen, halogen, linear or branchedC1-6 alkoxy, 2-morpholinoethoxy, C3-6 cycloalkyloxy, or C1-5 haloalkyloxy;
- R2 and R4 are independently selected from the group consisting of hydrogen or branched or linear C1-6 alkyl;
- R3 is selected from the group consisting of hydrogen, heterocycle or substituted heterocycle, or R6;
- R5 is selected from the group consisting of hydrogen, halogen, trifluoromethyl. -R6 is

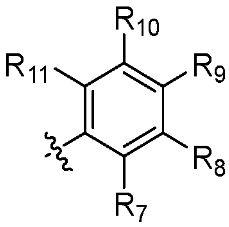

- R7,R8, R9, R10 andR11 are independently selected from the group consisting of hydrogen, halogen, linear or branchedC1-6 alkoxy .

In one embodiment, A is

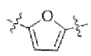.

In another preferred embodiment, A is

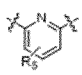.

In a further preferred embodiment, A is

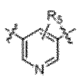.

In another preferred embodiment, A is

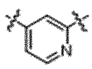.

In another preferred embodiment, A is selected from

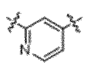, 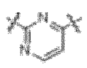 or 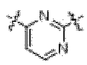.

In a further preferred embodiment, A is selected from the group consisting of

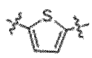 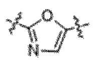 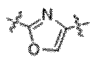 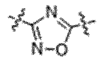 or 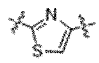 .

In one embodiment, R1 is selected from the group consisting of hydrogen, chloro, bromo, fluoro, methoxy, ethoxy, isopropoxy, propoxy, butoxy, 2-morpholinoethoxy, cyclopropoxy; cyclopentyloxy, cyclohexyloxy, cyclobutyloxy, or trifluoromethoxy. In a preferred embodiment, R1 is selected from the group consisting of hydrogen, chloro, methoxy, ethoxy, isopropoxy, propoxy, butoxy, 2-morpholinoethoxy, cyclopentyloxy, or trifluoromethoxy. In a further preferred embodiment, R1 is selected from the group consisting of chloro, methoxy, ethoxy, isopropoxy or 2-morpholinoethoxy. In a further preferred embodiment, R1 is selected from the group consisting of chloro, methoxy, ethoxy, isopropoxy, propoxy, butoxy, trifluoromethoxy or cyclopentyloxy. In a further preferred embodiment, R1 is methoxy.

In one embodiment, R2 andR4 are independently selected from the group consisting of hydrogen, methyl, ethyl, propyl, or isopropyl. In a preferred embodiment, R2 andR4 are independently selected from hydrogen or methyl. In a further preferred embodiment, R2 andR4 are each hydrogen. In a further preferred embodiment, R4 is selected from hydrogen or methyl.

In one embodiment, R3 is selected from the group consisting of hydrogen, thiophene, furan, 1H-pyrrol-2-yl,1-methyl-1H-pyrrol-2-yl, 1-ethyl-1H-pyrrol-2-yl,1H-imidazol-5-yl, 1H-pyrazol-5-yl, 2-oxazole, 2-thiazole, 5-oxazole, 5-thiazole, 1,3,4-oxadiazole, 1,3,4-thiadiazole, 3-isoxazole, 5-isoxazole, 3-isothiazole, 5-isothiazole, isoxazolpyridin-3-yl; pyridin-4-yl, pyridin-2-yl; 2-morpholinopyridin-3-yl, 4-((dimethylamino)methyl)thiophen-2-yl, orR6. In a preferred embodiment, R3 is selected from the group consisting of hydrogen, thiophene, pyridin-3-yl; pyridin-4-yl, pyridin-2-yl; 2-morpholinopyridin-3-yl, 4-((dimethylamino)methyl)thiophen-2-yl, orR6. In a further preferred embodiment, R3 is selected from the group consisting of thiophene, 4-((dimethylamino)methyl)thiophen-2-yl, pyridinyl, 2-morpholinopyridin-3-yl or R6. In a further preferred embodiment, R3 is selected from the group consisting of thiophene, 2-morpholinopyridin-3-yl or R6. In another preferred embodiment, R3 is thiophene or R6. In another preferred embodiment, R3 is R6.

In one embodiment, R5 is selected from the group consisting of hydrogen, chloro, bromo, fluoro, or trifluoromethyl. In a preferred embodiment, R5 is selected from the group consisting of hydrogen, fluoro or trifluoromethyl. In a further preferred embodiment, R5 is selected from hydrogen or fluorine.

In one embodiment R7, R8, R9, R10 andR11 are independently selected from the group consisting of hydrogen, chlorine, fluorine, bromine, methoxy, ethoxy, propoxy or isoproxy.

In a preferred embodiment, R7 is selected from hydrogen, chlorine, fluorine or methoxy. In a further preferred embodiment, R7 is hydrogen.

In a preferred embodiment, R8 andR10 are independently selected from hydrogen or methoxy.

In a preferred embodiment, R9 is hydrogen.

In a preferred embodiment, R11 is selected from the group consisting of hydrogen, chlorine or fluorine. In a further preferred embodiment, R11 is hydrogen.

In a preferred embodiment, the compound(s) of Formula (I) is selected from the group consisting of:

(*E*)-5-(4-chlorophenyl)-*N'*-(thiophen-2-ylmethylene)furan-2-carbohydrazide (Compound 1);
(E)-5-(4-chlorophenyl)-N'-(3,5-dimethoxybenzylidene)furan-2-carbohydrazide (Compound 2);
(*E*)-5-(4-ethoxyphenyl)-*N'*-(thiophen-2-ylmethylene)furan-2-carbohydrazide (Compound 3);
(*E*)-5-(4-ethoxyphenyl)-*N'*-((2-morpholinopyridin-3-yl)methylene)furan-2-carbohydrazide (Compound 4);
(*E*)-*N'*-((4-((dimethylamino)methyl)thiophen-2-yl)methylene)-5-(4-ethoxyphenyl)furan-2-carbohydrazide (Compound 5);
(*E*)-5-(4-ethoxyphenyl)-*N'*-(pyridin-3-ylmethylene)furan-2-carbohydrazide (Compound 6);
(*E*)-5-(4-isopropoxyphenyl)-*N'*-(thiophen-2-ylmethylene)furan-2-carbohydrazide (Compound 7);
(*E*)-*N'*-((4-((dimethylamino)methyl)thiophen-2-yl)methylene)-5-(4-isopropoxyphenyl)furan-2-carbohydrazide (Compound 8);
(*E*)-5-(4-(2-morpholinoethoxy)phenyl)-*N'*-(thiophen-2-ylmethylene)furan-2-carbohydrazide (Compound 9);
(*E*)-6-(4-chlorophenyl)-*N'*-(3,5-dimethoxybenzylidene)picolinohydrazide (Compound 10);
(*E*)-6-(4-chlorophenyl)-*N'*-(thiophen-2-ylmethylene)picolinohydrazide (Compound 11);
(*E*)-6-(4-ethoxyphenyl)-*N'*-(thiophen-2-ylmethylene)picolinohydrazide (Compound 12);
(*E*)-6-(4-isopropoxyphenyl)-*N'*-(thiophen-2-ylmethylene)picolinohydrazide (Compound 13);
(*E*)-*N'*-(3,5-dimethoxybenzylidene)-6-(4-methoxyphenyl)picolinohydrazide (Compound 14);
(*E*)-*N'*-(3,5-dimethoxybenzylidene)-6-(4-propoxyphenyl)picolinohydrazide (Compound 15);
(*E*)-5-(4-chlorophenyl)-*N'*-(3,5-dimethoxybenzylidene)nicotinohydrazide (Compound 16);
(*E*)-*N'*-(3,5-dimethoxybenzylidene)-5-(4-(trifluoromethoxy)phenyl)nicotinohydrazide (Compound 17);
(*E*)-*N'*-(3,5-dimethoxybenzylidene)-5-(4-ethoxyphenyl)nicotinohydrazide (Compound 18);
(*E*)-*N'*-(3,5-dimethoxybenzylidene)-5-(4-isopropoxyphenyl)nicotinohydrazide (Compound 19);

(*E*)-2-(4-chlorophenyl)-*N'*-(3,5-dimethoxybenzylidene)isonicotinohydrazide (Compound 20);
(*E*)-*N'*-(3,5-dimethoxybenzylidene)-2-(4-(trifluoromethoxy)phenyl)isonicotinohydrazide (Compound 21);
(*E*)-*N'*-(3,5-dimethoxybenzylidene)-2-(4-ethoxyphenyl)isonicotinohydrazide (Compound 22);
(*E*)-*N'*-(3,5-dimethoxybenzylidene)-2-(4-isopropoxyphenyl)isonicotinohydrazide (Compound 23);
(*E*)-*N'*-(3,5-dimethoxybenzylidene)-6-(4-isopropoxyphenyl)picolinohydrazide (Compound 24);
(*E*)-*N'*-(3,5-dimethoxybenzylidene)-5-(4-methoxyphenyl)nicotinohydrazide (Compound 25);
(*E*)-*N'*-(3,5-dimethoxybenzylidene)-5-(4-propoxyphenyl)nicotinohydrazide (Compound 26);
(*E*)-5-(4-butoxyphenyl)-*N'*-(3,5-dimethoxybenzylidene)nicotinohydrazide (Compound 27);
(*E*)-5-(4-(cyclopentyloxy)phenyl)-*N'*-(3,5-dimethoxybenzylidene)nicotinohydrazide (Compound 28);
(*E*)-*N'*-(3,5-dimethoxybenzylidene)-2-(4-methoxyphenyl)isonicotinohydrazide (Compound 29);
(*E*)-*N'*-(3,5-dimethoxybenzylidene)-2-(4-propoxyphenyl)isonicotinohydrazide (Compound 30);
(*E*)-*N'*-(3,5-dimethoxybenzylidene)-6-(4-ethoxyphenyl)picolinohydrazide (Compound 31);
(*E*)-6-(4-ethoxyphenyl)-*N'*-(2-fluorobenzylidene)picolinohydrazide (Compound 32);
(*E*)-*N'*-(2-chlorobenzylidene)-6-(4-ethoxyphenyl)picolinohydrazide (Compound 33);
(*E*)-*N'*-(2,6-dichlorobenzylidene)-6-(4-ethoxyphenyl)picolinohydrazide (Compound 34);
(*E*)-*N'*-(2-chloro-6-fluorobenzylidene)-6-(4-ethoxyphenyl)picolinohydrazide (Compound 35);
(*E*)-6-(4-ethoxyphenyl)-*N'*-(2-methoxybenzylidene)picolinohydrazide (Compound 36);
(*E*)-6-(4-ethoxyphenyl)-*N'*-((2-morpholinopyridin-3-yl)methylene)picolinohydrazide (Compound 37);
(*E*)-2-(4-butoxyphenyl)-*N'*-(3,5-dimethoxybenzylidene)isonicotinohydrazide (Compound 38);
(*E*)-2-(4-(cyclopentyloxy)phenyl)-*N'*-(3,5-dimethoxybenzylidene)isonicotinohydrazide (Compound 39);
(*E*)-*N'*-(3,5-dimethoxybenzylidene)-6-(4-(trifluoromethoxy)phenyl)picolinohydrazide (Compound 40);
(*E*)-6-(4-butoxyphenyl) -*N'*-(3,5-dimethoxybenzylidene)picolinohydrazide (Compound 41);
(*E*)-4-(4-chlorophenyl)-*N'*-(3,5-dimethoxybenzylidene)picolinohydrazide (Compound 42);
(*E*)-4-(4-chlorophenyl)-*N'*-(thiophen-2-ylmethylene)picolinohydrazide (Compound 43);
(*E*)-*N'*-(3,5-dimethoxybenzylidene)-4-(4-ethoxyphenyl)picolinohydrazide (Compound 44);
(*E*)-4-(4-ethoxyphenyl)-*N'*-(thiophen-2-ylmethylene)picolinohydrazide (Compound 45);
(*E*)-*N'*-(3,5-dimethoxybenzylidene)-4-(4-isopropoxyphenyl)picolinohydrazide (Compound 46);
(*E*)-4-(4-isopropoxyphenyl)-*N'*-(thiophen-2-ylmethylene)picolinohydrazide (Compound 47);
(*E*)-4-(4-(cyclopentyloxy)phenyl)-*N'*-(3,5-dimethoxybenzylidene)picolinohydrazide (Compound 48);
(*E*)-*N'*-(3,5-dimethoxybenzylidene)-4-(4-(trifluoromethoxy)phenyl)picolinohydrazide (Compound 49);
(*E*)-4-(4-(cyclopentyloxy)phenyl)-*N'*-(thiophen-2-ylmethylene)picolinohydrazide (Compound 50);
(*E*)-*N'*-(thiophen-2-ylmethylene)-4-(4-(trifluoromethoxy)phenyl)picolinohydrazide (Compound 51);
(*E*)-2-(4-chlorophenyl)-*N'*-(3,5-dimethoxybenzylidene)pyrimidine-4-carbohydrazide (Compound 52);
(*E*)-*N'*-(3,5-dimethoxybenzylidene)-2-(4-ethoxyphenyl)pyrimidine-4-carbohydrazide (Compound 53);
(*E*)-*N'*-(3,5-dimethoxybenzylidene)-2-(4-isopropoxyphenyl)pyrimidine-4-carbohydrazide (Compound 54);
(*E*)-2-(4-(cyclopentyloxy)phenyl)-*N'*-(3,5-dimethoxybenzylidene)pyrimidine-4-carbohydrazide (Compound 55) ;
(*E*)-*N'*-(3,5-dimethoxybenzylidene)-2-(4-(trifluoromethoxy)phenyl)pyrimidine-4-carbohydrazide (Compound 56);
(*E*)-*N'*-(3,5-dimethoxybenzylidene)-2-(4-methoxyphenyl)pyrimidine-4-carbohydrazide (Compound 57);
(*E*)-*N'*-(3,5-dimethoxybenzylidene)-2-(4-propoxyphenyl)pyrimidine-4-carbohydrazide (Compound 58);
(*E*)-2-(4-butoxyphenyl)-*N'*-(3,5-dimethoxybenzylidene)pyrimidine-4-carbohydrazide (Compound 59);
(*E*)-4-(4-chlorophenyl)-*N'*-(3,5-dimethoxybenzylidene)pyrimidine-2-carbohydrazide (Compound 60);
(*E*)-*N'*-(3,5-dimethoxybenzylidene)-4-(4-ethoxyphenyl)pyrimidine-2-carbohydrazide (Compound 61);
(*E*)-*N'*-(3,5-dimethoxybenzylidene)-4-(4-isopropoxyphenyl)pyrimidine-2-carbohydrazide (Compound 62);
(*E*)-4-(4-(cyclopentyloxy)phenyl)-*N'*-(3,5-dimethoxybenzylidene)pyrimidine-2-carbohydrazide (Compound 63);
(*E*)-*N'*-(3,5-dimethoxybenzylidene)-4-(4-(trifluoromethoxy)phenyl)pyrimidine-2-carbohydrazide (Compound 64);
(*E*)-*N'*-(3,5-dimethoxybenzylidene)-4-(4-methoxyphenyl)pyrimidine-2-carbohydrazide(Compound 65);
(*E*)-*N'*-(3,5-dimethoxybenzylidene)-4-(4-propoxyphenyl)pyrimidine-2-carbohydrazide (Compound 66);
(*E*)-4-(4-butoxyphenyl)-*N'*-(3,5-dimethoxybenzylidene)pyrimidine-2-carbohydrazide (Compound 67);
*(E)*-*N'*-(3,5-dimethoxybenzylidene)-6-(4-ethoxyphenyl)-4-fluoropicolinohydrazide (Compound 68);
(*E*)-*N'*-(3,5-dimethoxybenzylidene)-6-(4-ethoxyphenyl)-4-trifluoromethyl)picolinohydrazide (Compound 69);
(*E*)-*N'*-(3,5-dimethoxybenzylidene)-5-(4-ethoxyphenyl)-4-fluoronicotinohydrazide (Compound 70);
(*E*)-*N'*-(3,5-dimethoxybenzylidene)-5-(4-methoxyphenyl)furan-2-carbohydrazide (Compound 71);
(*E*)-*N'*-(3,5-dimethoxybenzylidene)-5-(4-methoxyphenyl)thiophene-2-carbohydrazide (Compound 72);
(*E*)-*N'*-(3,5-dimethoxybenzylidene)-2-(4-methoxyphenyl)oxazole-5-carbohydrazide (Compound 73);
(*E*)-*N'*-(3,5-dimethoxybenzylidene)-2-(4-methoxyphenol)oxazole-4-carbohydrazide (Compound 74);
(*E*)-*N'*-(3,5-dimethoxybenzylidene)-3-(4-methoxyphenol)-1,2,4-oxadiazole-5-carbohydrazide (Compound 75);
(*E*)-*N'*-(3,5-dimethoxybenzylidene)-2-(4-methoxyphenyl)thiazole-4-carbohydrazide (Compound 76);
(*E*)-*N'*-(3,5-dimethoxybenzylidene)-5-(4-methoxyphenyl)-1,2,4-thiadiazole-3-carbohydrazide (Compound 77);

(*E*)-5-(4-chlorophenyl)-*N'*-(3,5-dimethoxybenzylidene)-N-methylfuran-2-carbohydrazide (Compound 78);
(*E*)-*N'*-(3,5-dimethoxybenzylidene)-6-(4-ethoxyphenyl)-*N*-methylpicolinohydrazide (Compound 79).

In one embodiment, the Formula (I) compound (s) are selective inhibitors of the voltage-gated sodium channels Nav 1.7 and/or Nav 1.8. In a preferred embodiment, the compound(s) Formula (I) are dual inhibitors of the voltage-gated sodium channels Nav 1.7 and Nav 1.8.

In some embodiments, the compound(s) of Formula (I) may be basic in nature, and accordingly pharmaceutically acceptable salts may be obtained by the addition of organic or inorganic acids. Non-limiting examples of organic acids that may be used are fumaric, maleic, benzoic, lactic acids, among others. Among the inorganic acids may be mentioned hydrochloric acid, phosphoric acid, hydrobromic acid, sulfuric acid, nitric acid, among others.

In some embodiments, the compound (s) of Formula (I) may be obtained in the form of crystals, which may optionally be presented as pharmaceutically acceptable solvates, wherein the solvent is incorporated in stoichiometric proportions or not into the crystal lattice. In further embodiments, the crystallization solvent is water, resulting in pharmaceutically acceptable hydrates.

Finally, in some embodiments, the compound(s) of Formula (I) may exhibit more than one isomer, including without limitation, spatial isomerism such as geometric and optical isomerism.

## Definitions

For the purpose of clarity or elucidation of the terms used in the present invention, the following definitions are presented, and the scope is not limited thereto.

The term "straight or branched C1-6 alkyl" refers to saturated straight or branched chain hydrocarbons such as methyl, ethyl, propyl, butyl, isopropyl, *tert-butyl,* pentyl, hexyl, not limited thereto.

The term "linear or branchedC1-6 alkoxy" refers to alkyl groups bonded to a radical oxygen, including methoxy, ethoxy, propoxy, butoxy, pentoxy, hexoxy, isoproxy, isobutoxy, *tert*-butoxy, and is not limited thereto.

The term "C1-6haloalkyloxy" refers to alkoxy groups bonded to a halogen, including groups such as trifluoromethoxy, difluoromethoxy, fluoromethoxy, chloromethoxy, dichloromethoxy, trichloromethoxy, 2,2,2-trifluoroethoxy, 2,2-difluoroethoxy, 2-fluoroethoxy, 2-chloroethoxy, 2,2-dichloroethoxy, 2,2,2-trichloroethoxy, 3,3,3-trifluoropropoxy, 3,3-difluoropropoxy, 3-fluoroproxy, 3-chloropropoxy, 3,3-dichloropropoxy, 3,3,3-trichloropropoxy, 4,4,4-trifluorobutoxy, 5,5,5-trifluoropentoxy, not limited thereto.

The term "C3-6 cycloalkyloxy" refers to groups such as cyclopropoxy; cyclopentyloxy; cyclohexyloxy; cyclobutyloxy, not limited thereto.

The term "heterocycle" or "substituted heterocycle" refers to groups such as thiophene, furan, 1H-pyrrol-2-yl, 1-methyl-1H-pyrrol-2-yl,1-ethyl-1H-pyrrol-2-yl, 1H-imidazol-5-yl,1H-pyrazol-5-yl, 2-oxazole, 2-thiazole, 5-oxazole, 5-thiazole, 1,3,4-oxadiazole, 1,3,4-thiadiazole, 3-isoxazole, 5-isoxazole, 3-isothiazole, 5-isothiazole, isoxazolpyridin-3-yl; pyridin-4-yl, pyridin-2-yl; 2-morpholinopyridin-3-yl, 4-((dimethylamino)methyl)thiophen-2-yl, not limited thereto.

In a second embodiment, the present invention features a composition comprising a therapeutically effective amount of compound(s) of Formula (I) of the present invention or a pharmaceutically acceptable salt, hydrate, solvate, and isomerthereof; and one or more pharmaceutically acceptable excipients.

Pharmaceutically acceptable excipients are considered to be any substance, other than the active pharmaceutical ingredient, that has been evaluated for safety and that is intentionally added to the dosage form. Such excipients are selected according to the pharmaceutical dosage form of interest, its route of administration, physicochemical compatibility with the active ingredient, and the effect on efficacy.

Furthermore, said excipients are widely known in the state of art and are classified according to their function, including without limitation diluents, binders, disintegrants or disaggregants, lubricants, suspending agents, thickeners, solvents, surfactants, glidants, anti-caking or flowing agents, coating agents, plasticizers, sweeteners, sweeteners, isotonicity agents, dyes and pigments, preservatives, antioxidants, pH modifying or control agents, complexing agents, chelating agents, flavoring, flavoring, viscosity modifying agents, opacifiers, permeation promoters, among others.

The pharmaceutical compositions may be administered by various routes including, but not limited to, oral, sublingual, nasal, parenteral, injectable, submuscular, topical, transdermal, ocular, rectal.

In a third embodiment, the present invention features the use of compound(s) of Formula (I) or a pharmaceutically acceptable salt, hydrate, solvate, and isomer thereof to prepare a medicament for treating neuropathic pain-related pathologies.

In a preferred embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention features a method of treating, preventing, alleviating, suppressing,

and/or controlling neuropathic pain-related pathologies comprising administering an effective amount of compound(s) of Formula (I) or a pharmaceutically acceptable salt, hydrate, solvate, and isomer thereof. In a preferred embodiment, the method is for the treatment, prevention, alleviation, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia. In a further preferred embodiment, the administration of the at least one compound of Formula (I) is selected from the group comprising oral, sublingual, nasal, parenteral, injectable, submuscular, topical, transdermal, ocular, and rectal.

In a fifth embodiment of the invention, there are provided processes of obtaining compound(s) of Formula (I) comprising the following steps:

(a) Forming the intermediate of Formula III:

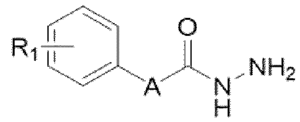

Formula III

from the hydrazinolysis reaction of an intermediate of Formula IV:

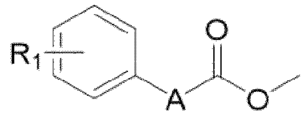

Formula IV

(b) obtaining a Formula I compound wherein R4 is hydrogen;

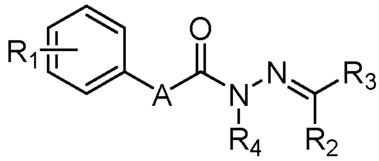

Formula I

from condensation of intermediates of Formula II:

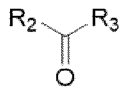

Formula II:

and Formula III, with or without presence of catalyst and a suitable solvent; wherein, A, R1, R2, R3, R5, R6, R7, R8, R9, R10, R11 are independently selected from the groups previously described.

In one embodiment, the process further comprises a step (c) of forming compound(s) of Formula (I) wherein R4 is linear or branched C1-6 alkyl from the nucleophilic substitution reaction of a compound obtained in (b), with linear or branched C1-6 alkyl halides in the presence of inorganic base and polar aprotic solvents.

In one embodiment of the process, in step (b) said catalyst is selected from concentrated hydrochloric acid, acetic acid, trifluoroacetic acid, formic acid, or combinations thereof and said solvent is selected from dimethylformamide, alcohols, or combinations thereof. In another embodiment, in step (c) said inorganic base may be selectedfrom K2CO3 or NaH.

The compound(s) of Formula (I) of the present invention were prepared from the synthetic route described in General Scheme 1. However, those skilled in the art will readily appreciate that additional detailing and/or modifications to the arrangement of one or more steps may be performed without departing from the processes taught herein. Such variations may be, without limitation, combinations of solvents and catalysts, including stereoselectives, protecting groups, among others.

In the following General Scheme 1, the formation of the intermediates of Formula II, III and IV is described, in addition to the formation of the compound(s) of Formula (I), wherein Formula Ia corresponds to compounds in whichR4 is hydrogen and Formula Ib corresponds to compounds in whichR4 is a C1-6 alkylgroup (represented by R in the scheme), from these intermediates.

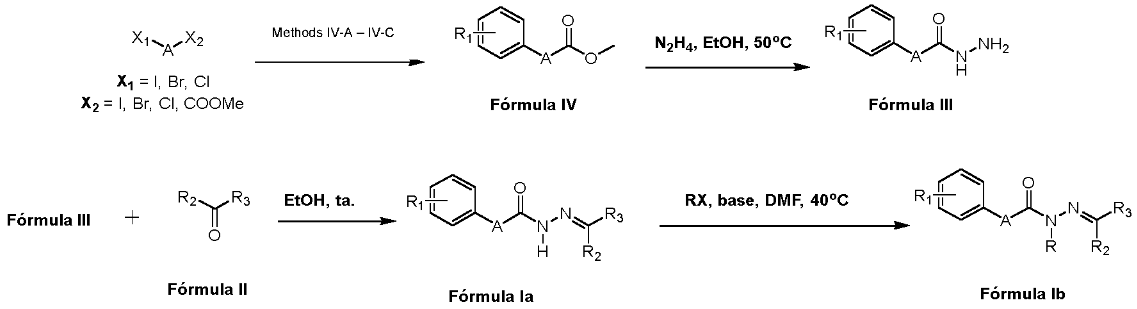


## General Scheme 1

As illustrated in General Scheme 1, compounds of Formula Ia can be prepared from condensation reactions of the intermediates of Formula III with the intermediates of Formula II, without or upon acid catalysis using concentrated hydrochloric acid, acetic acid, trifluoroacetic acid or formic acid in suitable solvents such as DMF or alcohols such as methanol, ethanol or propanol. Compounds of Formula Ib (R = R4 being C1-6 alkyl group), can be obtained from an additional step, starting from compounds of Formula Ia, by nucleophilic substitution reactions with corresponding alkyl halides, in the presence of an inorganic base such as K2CO3 or NaH and polar aprotic solvents. In turn, intermediates of Formula III can be prepared by hydrazinolysis reaction from the corresponding esters (Formula IV) obtained following methods IV-A to IV-C. Intermediates of Formula II can be obtained commercially. The details of such methodologies are described below.

**<u>Obtaining Compounds of Formula IV:</u>**

**Method IV-A:**

*Intermediates IV-1 - IV-33 / IV-52 - IV-59*

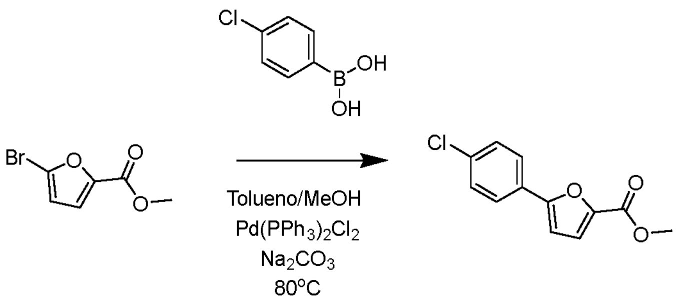


Into a 25-mL round-bottom flask were added 1 mmol of methyl 5-bromofuran-2-carboxylate, 1.5 mmol of 4-chlorophenylboronic acid, and 0.075 mmol of Pd(PPh3)2Cl2. 5 mL of toluene and 5 mL MeOH were added. Once the reactants dissolved, 20 mmol Na2CO3 was added as a 2M solution in distilled water. Subsequently, the reaction mixture was allowed to stir at 80 °C. After total consumption of the starting reagent (monitored by CCF), the reaction mixture was filtered on *Celite* and concentrated. Subsequently, the resulting oil was dissolved with distilled water (25 mL) and extracted with AcOEt (3 x 25 mL). The organic phases were pooled, dried over anhydrous magnesium sulfate, filtered and concentrated to give the product, which was purified by column chromatography(n-hexane/AcOEt: 100/0 → 60/40) (Table 1).

For compounds IV-17 through IV-28, the solvent was replaced with dioxane, the catalyst used was Pd(PPh3)4 , and the temperature was maintained at 110 oC for 12 hours (Table 1).

For compounds IV-29 to IV-33 the solvent was replaced with dioxane and the temperature was maintained at

110 oC for 12 hours (Table 1).

**Table 1.** Intermediates obtained, from the corresponding reagents, following Method IV-A.

| Intermediate | Structure | Intermediate | Structure |
|---|---|---|---|
| IV-1 | 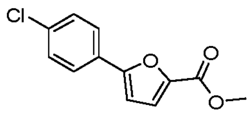 | IV-22 | 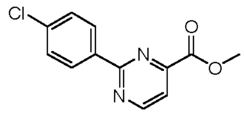 |
| IV-2 | 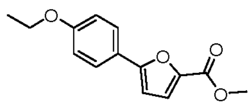 | IV-23 | 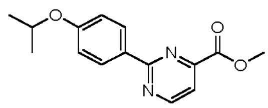 |
| IV-3 | 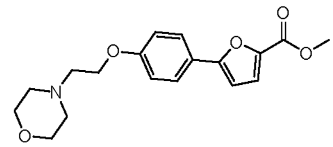 | IV-24 | 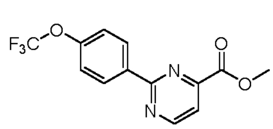 |
| IV-4 | 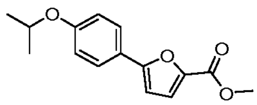 | IV-25 | 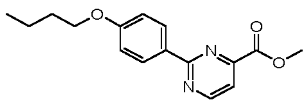 |
| IV-5 | 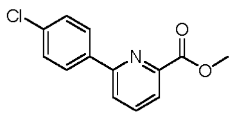 | IV-26 | 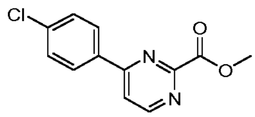 |
| IV-6 | 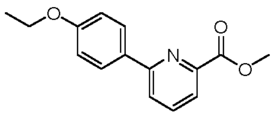 | IV-27 | 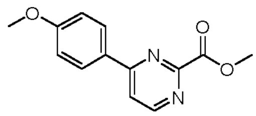 |
| IV-7 | 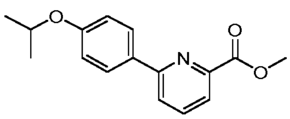 | IV-28 | 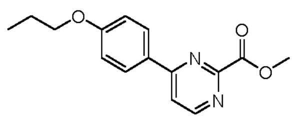 |
| IV-8 | 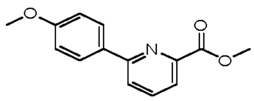 | IV-29 | 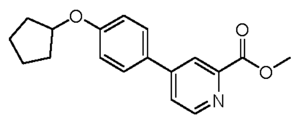 |
| IV-9 | 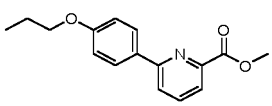 | IV-30 | 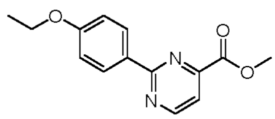 |
| IV-10 | 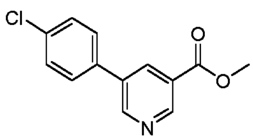 | IV-31 | 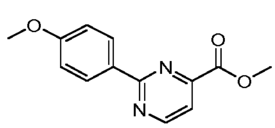 |
| IV-11 | 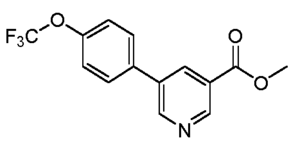 | IV-32 | 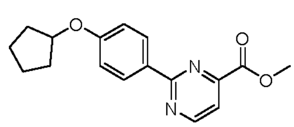 |

(continued)

| Intermediate | Structure | Intermediate | Structure |
|---|---|---|---|
| IV-12 | 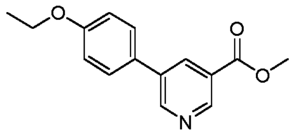 | IV-33 | 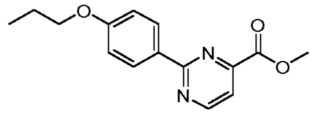 |
| IV-13 | 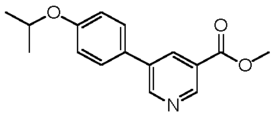 | IV-52 | 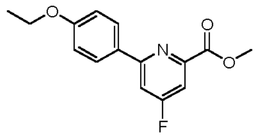 |
| IV-14 | 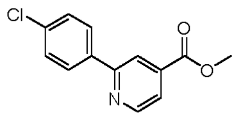 | IV-53 | 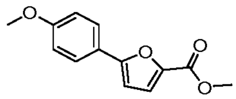 |
| IV-15 | 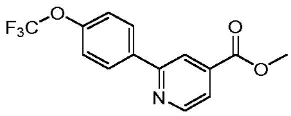 | IV-54 | 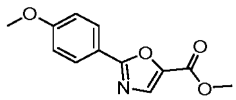 |
| IV-16 | 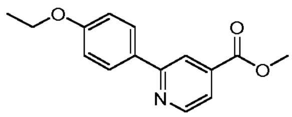 | IV-55 | 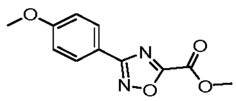 |
| IV-17 | 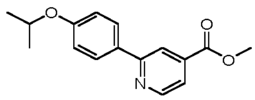 | IV-56 | 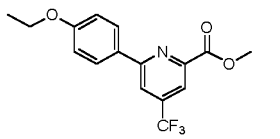 |
| IV-18 | 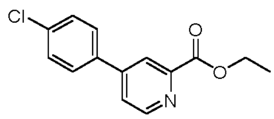 | IV-57 | 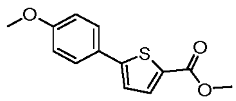 |
| IV-19 | 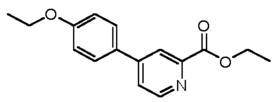 | IV-58 | 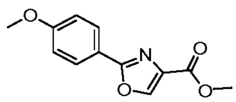 |
| IV-20 | 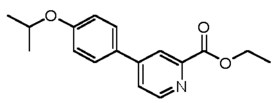 | IV-59 | 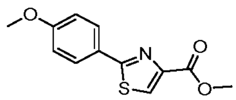 |
| IV-21 | 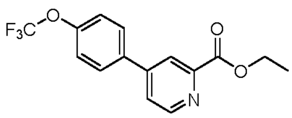 | | |

**Method IV-B:**

*Intermediates IV-34 - IV-43*

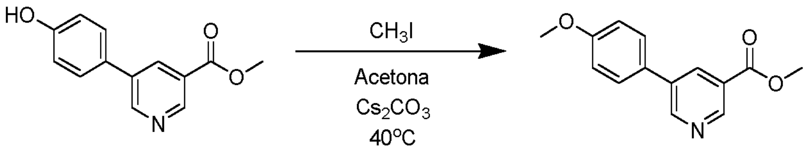


Into a 25-mL flask, 1.3 mmol of methyl 5-(4-hydroxyphenyl)nicotinate, 3 equivalents of cesium carbonate, and 9 mL of acetone were added. Subsequently, 2 equivalents of methyl iodide in 1 mL of acetone were added. The reaction mixture was maintained under stirring at 40 °C. After total consumption of the starting reagent (monitored by CCF), the reaction mixture was diluted with H2O (25 mL) and extracted with AcOEt (3 x 25 mL). The organic phases were pooled, dried over anhydrous magnesium sulfate, filtered and concentrated. Purification was performed by column chromatography(n-Hexane/AcOEt: 90/10 → 70/30) affording the intermediate of interest (Table 2).

Table 2. Intermediates obtained, from the corresponding reagents, following Method IV-B.

| Intermediate | Structure |
|---|---|
| IV-34 | 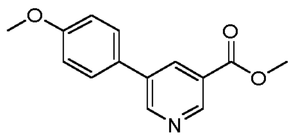 |
| IV-35 | 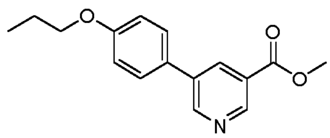 |
| IV-36 | 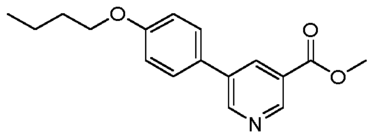 |
| IV-37 | 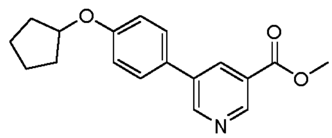 |
| IV-38 | 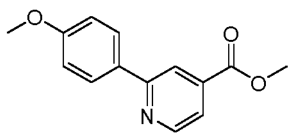 |
| IV-39 | 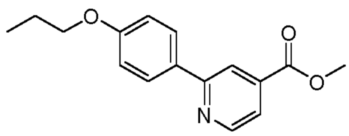 |
| IV-40 | 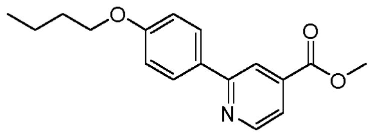 |
| IV-41 | 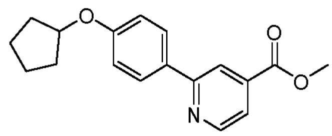 |

(continued)

| Intermediate | Structure |
|---|---|
| IV-42 | 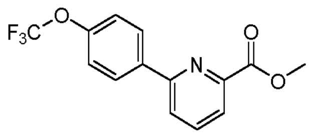 |
| IV-43 | 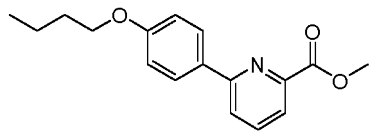 |

**Method IV-C:**

For the synthesis of intermediates IV-44 to IV-51 (Table 4), the synthesis of precursors IV-44i - IV-51i is required, which is obtained through two steps (IV-C-i and IV-C-ii), as described below.

Step IV-C-i

*Precursors IV-44i - IV-51i*

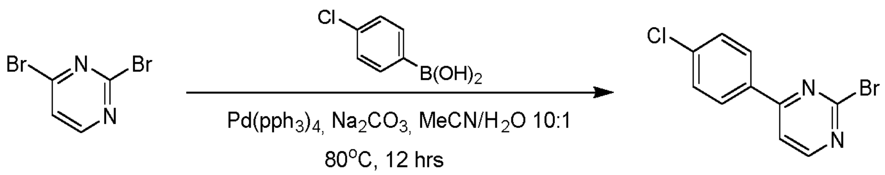


Into a round bottom flask were added 12.6 mmol of 2,4-dibromopyrimidine, 12.8 mmol of 2,4-dibromopyrimidine, 1.26 mmol ofPd(dppf)Cl2CH2Cl2, 25.2 mmol of Na2CO3 20 mL of MeCN and 2 mL of H2O. Subsequently, the reaction mixture was allowed to stir at 80 °C. After total consumption of the starting reagent (monitored by CCF), the reaction mixture was diluted with distilled water (30 mL) and extracted with AcOEt (3 x 10 mL). The organic phases were pooled, dried over anhydrous magnesium sulfate, filtered and concentrated to give the product, which was purified by column chromatography (n-hexane/AcOEt: 3/1 → 0/1) (Table 3).

For compound IV-47i, the solvent was replaced with a 10:1 dioxane/H2Omixture, using as catalyst Pd(dppf)Cl2CH2Cl2.

For compounds IV-48i to IV-51i, the starting reagent used was 2,4-dichloropyrimidine and the solvent a 10:1 dioxane/H2O mixture. Additionally, as catalyst was used Pd(PPh3)4 and as base K2CO3.

Table 3. Precursors obtained, from the corresponding reagents, following step IV-C-i of Method IV-C.

| Precursor | Structure | Precursor | Structure |
|---|---|---|---|
| IV-44i | 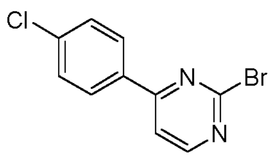 | IV-48i | 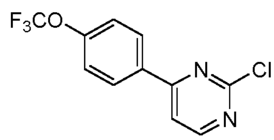 |
| IV-45i | 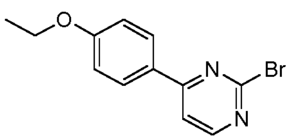 | IV-49i | 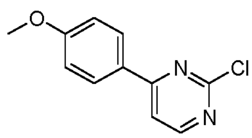 |

(continued)

| Precursor | Structure | Precursor | Structure |
|---|---|---|---|
| IV-46i | 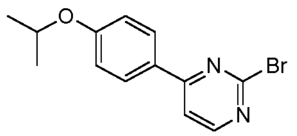 | IV-50i | 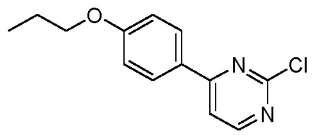 |
| IV-47i | 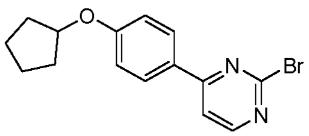 | IV-51i | 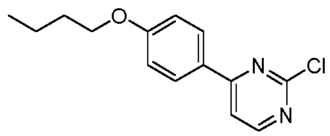 |

Step IV-C-ii

*Intermediates IV-44 - IV-51*

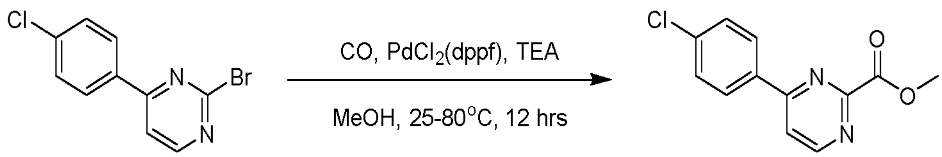


In a round bottom flask containing 7.4 mmol of 2-bromo-4-(4-chlorophenyl)pyrimidine in 5 mL of MeOH, 14.8 mmol of tea and 371 μmol ofPdCl2(dppf) were added. Subsequently, the reaction mixture degassed, purged with CO and maintained, under CO atmosphere (50 Psi), stirring at 80 °C for 12 hours. After total consumption of the starting reagent (monitored by CCF), the reaction mixture concentrated to obtain the product, which was purified by column chromatography(n-hexane/AcOEt: 3/1 → 0/1) (Table 4).

**Table 4.** Precursors obtained, from the corresponding reagents, following step IV-C-i of Method IV-C.

| Intermediate | Structure | Intermediate | Structure |
|---|---|---|---|
| IV-44 | 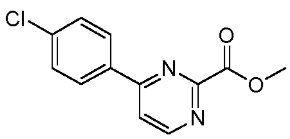 | IV-48 | 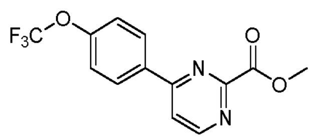 |
| IV-45 | 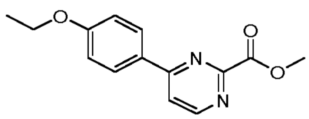 | IV-49 | 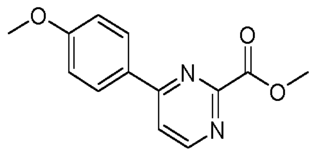 |
| IV-46 | 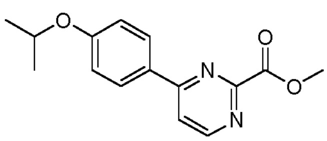 | IV-50 | 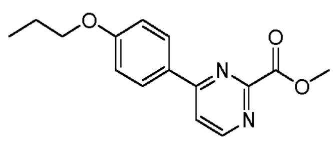 |
| IV-47 | 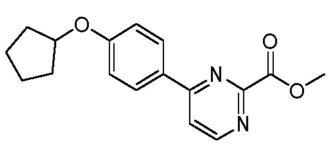 | IV-51 | 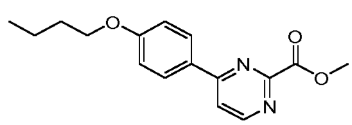 |

**Obtaining Compounds of Formula IV:**

*Intermediates III-1 - III-59*

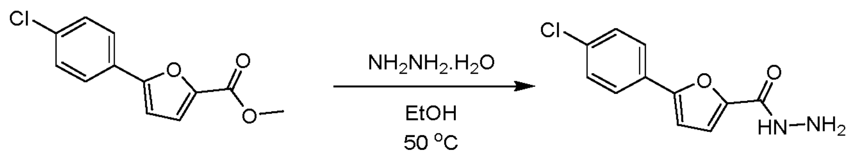


In a flask containing 8.8 mmol of methyl 5-(4-chlorophenyl)furan-2-carboxylate (intermediate IV-1), dissolved in 15 mL of ethanol, 3 equivalents of hydrazine hydroxide were added. The reaction medium was then subjected to heating to 50 °C. When total consumption of starting ester by CCF was detected, the solvent was concentrated to half volume under reduced pressure, and 25 mL of cold distilled water was added, leading to precipitation of a solid which was filtered and washed with 25 mL of cold distilled water and 15 mL of n-hexane, providing the hydrazide of interest (Table 5).

Table 5. Intermediates obtained, from the corresponding reagents, following the method described in obtaining the compounds of Formula III.

| Intermediate | Structure | Intermediate | Structure |
|---|---|---|---|
| III-1 | 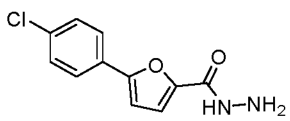 | III-30 | 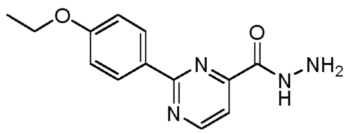 |
| III-2 | 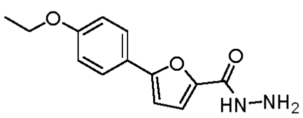 | III-31 | 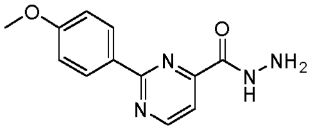 |
| III-3 | 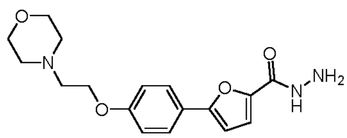 | III-32 | 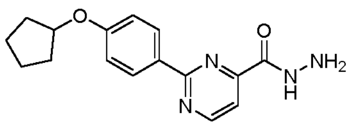 |
| III-4 | 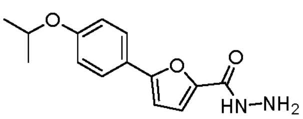 | III-33 | 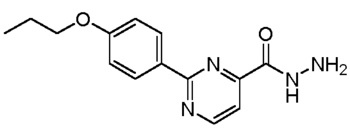 |
| III-5 | 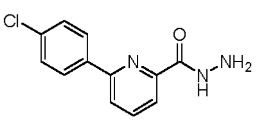 | III-34 | 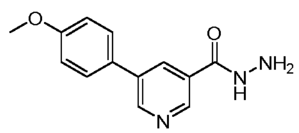 |
| III-6 | 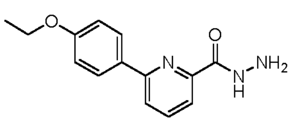 | III-35 | 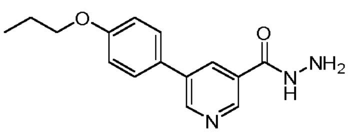 |
| III-7 | 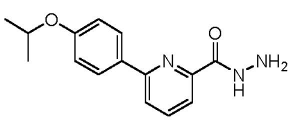 | III-36 | 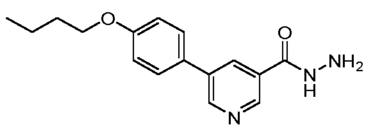 |

(continued)

| Intermediate | Structure | Intermediate | Structure |
|---|---|---|---|
| III-8 | 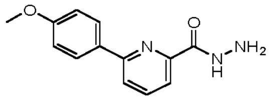 | III-37 | 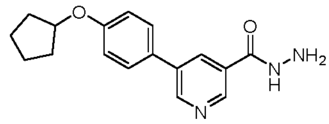 |
| III-9 | 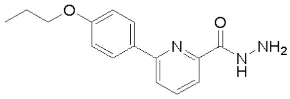 | III-38 | 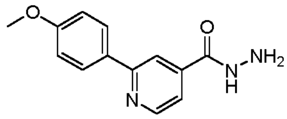 |
| III-10 | 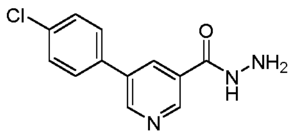 | III-39 | 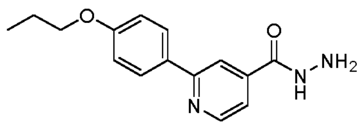 |
| III-11 | 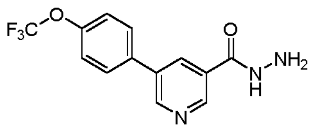 | III-40 | 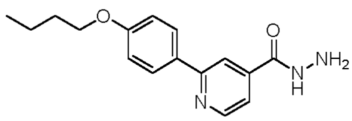 |
| III-12 | 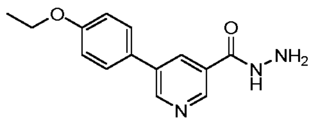 | III-41 | 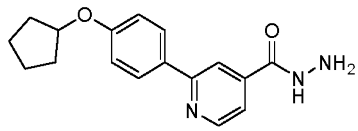 |
| III-13 | 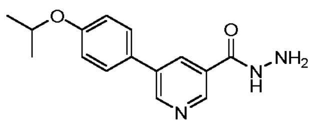 | III-42 | 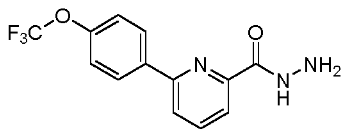 |
| III-14 | 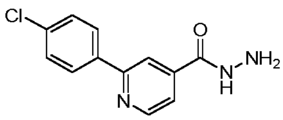 | III-43 | 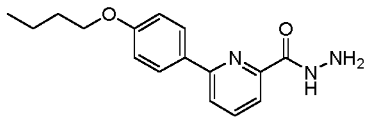 |
| III-15 | 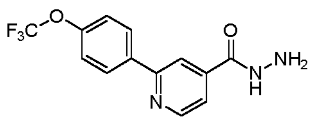 | III-44 | 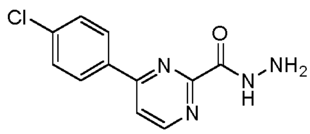 |
| III-16 | 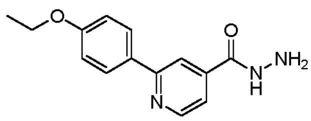 | III-45 | 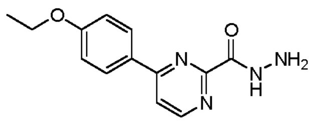 |
| III-17 | 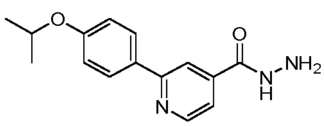 | III-46 | 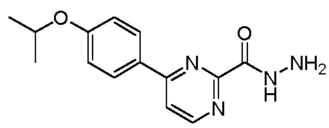 |
| III-18 | 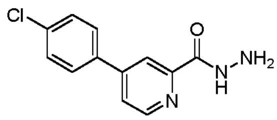 | III-47 | 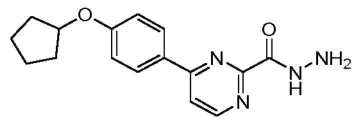 |

(continued)

| Intermediate | Structure | Intermediate | Structure |
|---|---|---|---|
| III-19 | 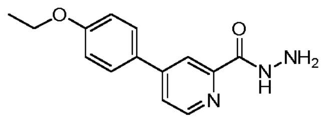 | III-48 | 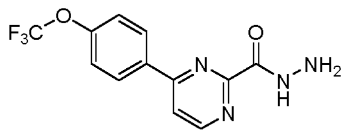 |
| III-20 | 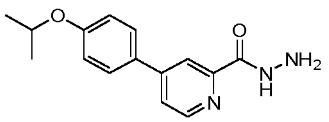 | III-49 | 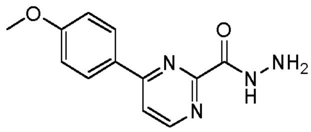 |
| III-21 | 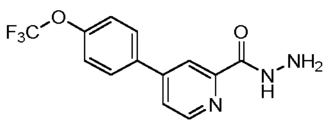 | III-50 | 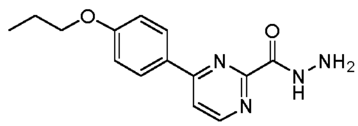 |
| III-22 | 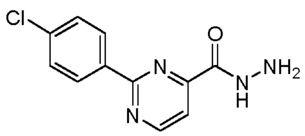 | III-51 | 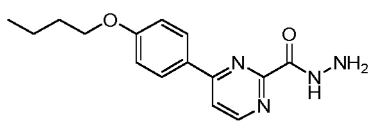 |
| III-23 | 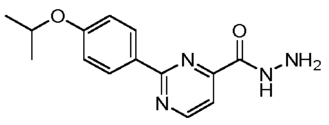 | III-52 | 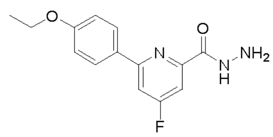 |
| III-24 | 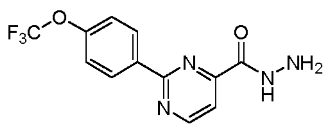 | III-53 | 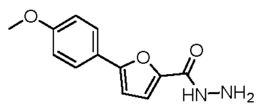 |
| III-25 | 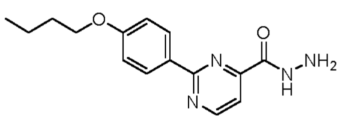 | III-54 | 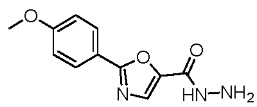 |
| III-26 | 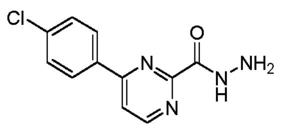 | III-55 | 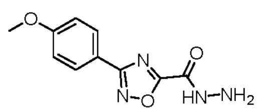 |
| III-27 | 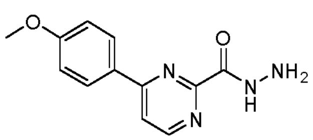 | III-56 | 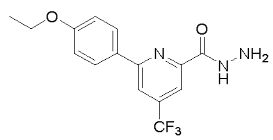 |
| III-28 | 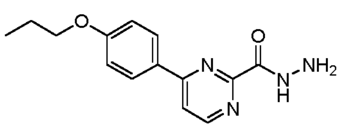 | III-57 | 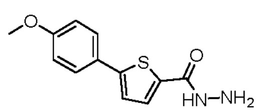 |
| III-29 | 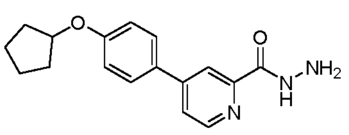 | III-58 | 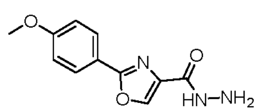 |

(continued)

| Intermediate | Structure | Intermediate | Structure |
|---|---|---|---|
| | | III-59 | 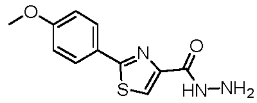 |

## Examples

The following examples, described in detail, serve to illustrate embodiments of the present invention without, however, having any limiting character to the scope of protection thereof.

The compound (s) of Formula (I) of the present invention were obtained from the condensation of the intermediates of Formula III, obtained according to previously described methodologies without being limited thereto, with commercial aldehydes of Formula II, according to General Scheme 1.

### Example 1. Obtaining Compounds of Formula (I):

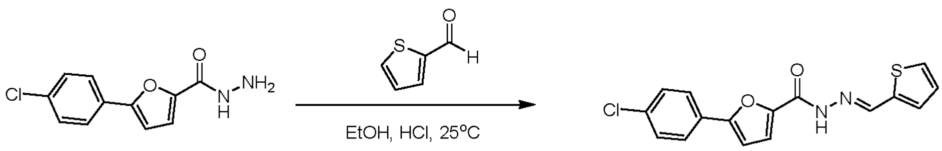


In a flask containing 1 mmol of 5-(4-chlorophenyl)furan-2-carbohydrazide (intermediate III-1), dissolved in 10 mL of ethanol, catalytic amounts of concentrated HCl were added. Subsequently, 1.1 mmol of thiophene-2-carbaldehyde dissolved in 2 mL of ethanol was added. The reaction medium remained under agitation until total consumption of the starting material (detected by CCF). After concentration to half the volume of the reaction medium under reduced pressure, 10 mL of ice-cold distilled water was added while keeping the mixture in an ice bath. After filtration and washing with 15 mL of ice water and 15 mL of hexane, compound 1 was obtained (Table 6).

Table 6. Compounds obtained, from the corresponding reagents, following the method described for formation of Compound 1.

| Compound No. | Structure | 1H-NMRI MS m/z [M+H] + |
|---|---|---|
| 1 | 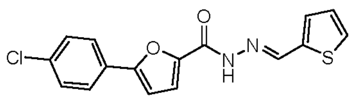 (*E*)-5-(4-chlorophenyl) -*N'*-(thiophen-2-ylmethylene)furan-2-carbohydrazide (Compound 1) | 400 MHz, DMSO-d6 δ 11,79 (s, 1H), 8,74 (s, 1H), 7,96 (d, 2H), 7,69 (d, 1H), 7,56 (d, 2H), 7,49 (d, 1H), 7,37 (d, 1H), 7,22 (d, 1H), 7,16 (t, 1H). [M+H]+: 331,7. |
| 2 | 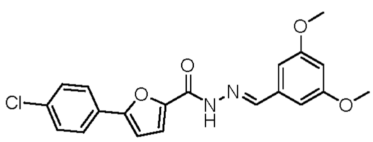 (*E*)-5-(4-chlorophenyl)-*N'*-(3,5-dimethoxybenzylidene)furan-2-carbohydrazide | 400 MHz, DMSO-*d6* *δ* 11,83 (s, 1H), 8,44 (s, 1H), 7,98 (d, *J* = 7,1 Hz, 2H), 7,58 (d, *J* = 8,3 Hz, 2H), 7,40 (s, 1H), 7,24 (d, *J* = 2,7 Hz, 1H), 6,89 (s, 2H), 6,59 (s, 1H), 3,80 (s, 6H). |

(continued)

| Compound No. | Structure | 1H-NMRI MS m/z [M+H] + |
|---|---|---|
| 3 | 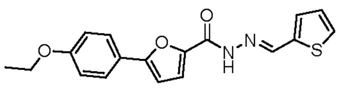 (*E*)-5-(4-ethoxyphenyl) -*N'*-(thiophen-2-ylmethylene)furan-2-carbohydrazide | 400 MHz, DMSO-*d6* $\delta$ 11,70 (s, 1H), 8,73 (s, 1H), 7,85 (d, *J* = 6,6Hz, 2H), 7,68 (d, *J* = 5,0 Hz, 1H), 7,48 (d, *J* = 3,3 Hz, 1H), 7,33 (s, 1H), 7,16 (dd, *J* = 5,0, 3,6 Hz, 1H), 7,06 - 7,01 (m, 3H), 4,11 - 4,07 (m, 1H), 1,35 (t, *J* = 5,0 Hz, 6H). [M+H]+: 341,5. |
| 4 | 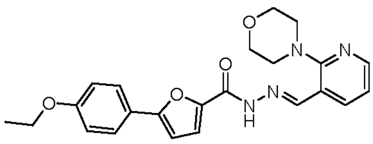 (*E*)-5-(4-ethoxyphenyl) -*N'*-((2-morpholinopyridin-3-yl)methylene)furan-2-carbohydrazide | 400 MHz, DMSO-*d6* $\delta$ 11.84 (s, 1H), 8.57 (s, 1H), 8.29 (dd, *J* = 4.9, 1.9 Hz, 1H), 8.10 (dd, *J* = 7.6, 1.9 Hz, 1H), 7.82 (d, *J* = 8.2 Hz, 2H), 7.34 (s, 1H), 7.09 (dd, *J* = 7.7, 4.7 Hz, 1H), 7.04 - 6.97 (m, 3H), 4.05 (q, *J* = 6.9 Hz, 2H), 3.77 (t, *J* = 4.5 Hz, 4H), 3.11 (t, *J* = 4.6 Hz, 4H), 1.31 (t, *J* = 6.9 Hz, 3H). |
| 5 | 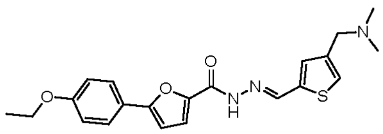 (*E*) -*N'*-((4-((dimethylamino)methyl)thiophen-2-yl)methylene)-5-(4-ethoxyphenyl)furan-2-carbohydrazide | 400 MHz, DMSO-*d6* $\delta$ 8.78 (s, 1H), 7.96 (d, *J* = 8.2 Hz, 2H), 7.57 - 7.37 (m, 3H), 7.12 (dd, *J* = 15.6, 5.9 Hz, 3H), 4.19 (q, *J* = 6.9 Hz, 2H), 3.47 (m, 2H), 2.25 (s, 6H), 1.45 (t, *J* = 6.9 Hz, 3H). [M+H]+: 398,4. |
| 6 | 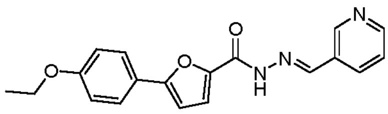 (*E*)-5-(4-ethoxyphenyl) -*N'*-(pyridin-3-ylmethylene)furan-2-carbohydrazide | 400 MHz, DMSO-*d6* $\delta$ 11.89 (s, 1H), 8.83 (s, 1H), 8.59 (d, *J* = 4.8 Hz, 1H), 8.52 (s, 1H), 8.12 (d, *J* = 8.0 Hz, 1H), 7.83 (s, 2H), 7.47 (dd, *J* = 7.9, 4.7 Hz, 1H), 7.35 (s, 1H), 7.01 (d, *J* = 8.7 Hz, 3H), 4.06 (q, *J* = 7.0 Hz, 2H), 1.31 (t, *J* = 7.0 Hz, 3H). |
| 7 | 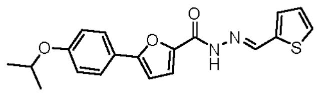 (*E*)-5-(4-isopropoxyphenyl) -*N'*-(thiophen-2-ylmethylene)furan-2-carbohydrazide | 500MHz, DMSO-*d6* $\delta$ 11,70 (s, 1H), 8,73 (s, 1H), 7,85 (d, *J* = 6,6 Hz, 2H), 7,68 (d, *J* = 5,0 Hz, 1H), 7,48 (d, *J* = 3,3 Hz, 1H), 7,33 (s, 1H), 7,16 (dd, *J* = 5,0, 3,6 Hz, 1H), 7,04 - 6,99 (m, 3H), 4,72 - 4,68 (m, 1H), 1,29 (d, *J* = 6,0 Hz, 6H). [M+H]+: 355,5. |
| 8 | 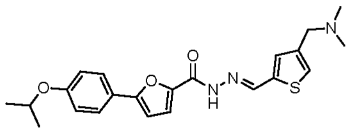 (*E*)-*N'*-((4-((dimethylamino)methyl)thiophen-2-yl)methylene)-5-(4-isopropoxyphenyl) furan-2-carbohydrazide | 400 MHz, DMSO-*d6* $\delta$ 11,71 (s, 1H), 8,67 (s, 1H), 7,85 (d, J = 7,8 Hz, 2H), 7,42 (s, 1H), 7,37 (s, 1H), 7,33 (s, 1H), 7,04 - 6,98 (m, 3H), 4,70 (sep, J = 6,1 Hz, 1H), 3,38 (s, 2H), 2,15 (s, 6H), 1,29 (d, J = 6,0 Hz, 6H). [M+H]+: 412,6. |
| 9 | 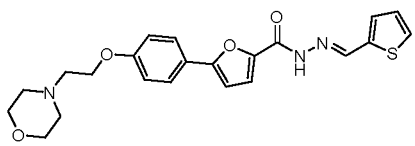 (*E*)-5-(4-(2-morpholinoethoxy) phnyl)-*N'*-(thiophen-2-ylmethylene)furan-2-carbohydrazide | 400 MHz, DMSO-*d6* $\delta$ 11.79 (s, 1H), 8.81 (s, 1H), 8.01 - 7.84 (m, 2H), 7.77 (d, *J* = 5.0 Hz, 1H), 7.56 (d, *J* = 3.6 Hz, 1H), 7.41 (s, 1H), 7.24 (t, *J* = 4.4 Hz, 1H), 7.15 (d, *J* = 8.4 Hz, 2H), 7.10 (d, *J* = 3.5 Hz, 1H), 4.23 (t, *J* = 5.7 Hz, 2H), 3.67 (t, *J* = 4.7 Hz, 4H), 2.79 (t, *J* = 5.6 Hz, 2H). |

(continued)

| Compound No. | Structure | 1H-NMRI MS m/z [M+H] + |
|---|---|---|
| 10 | 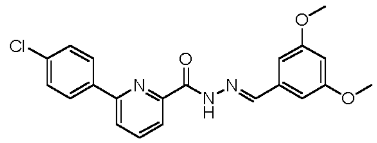 (*E*)-6-(4-chlorophenyl) -*N*'-(3,5-dimethoxybenzylidene)picolinohydrazide | 400 MHz, DMSO-*d*6 δ 11,95 (s, 1H), 8,66 (s, 1H), 8,39 (d, 2H), 8,24 (d, 1H), 8,13 (t, 1H), 8,07 (d, 1H), 7,59 (d, 2H), 6,92 (s, 2H), 6,60 (s, 1H), 3,81 (s, 6H). [M+H]+: 396,7. |
| 11 | 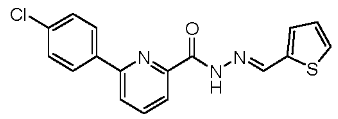 (*E*)-6-(4-chlorophenyl)-*N'*-(thiophen-2-ylmethylene)picolinohydrazide | 400 MHz, DMSO-*d*6 δ 11,92 (s, 1H), 8,96 (s, 1H), 8,40 (d, 2H), 8,24 (d, 1H), 8,13 (t, 1H), 8,08 (d, 1H), 7,71 (d, 1H), 7,59 (d, 2H), 7,50 (d, 1H), 7,17 (t, 1H). [M+H]+: 342,0. |
| 12 | 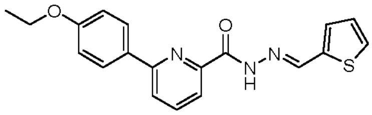 (*E*)-6-(4-ethoxyphenyl) -*N*'-(thiophen-2-ylmethylene)picolinohydrazide | 400 MHz, DMSO-*d*6 δ 11,88 (s, 1H), 8,95 (s, 1H), 8,30 (d, 2H), 8,13 (d, 1H), 8,05 (t, 1H), 7,97 (d, 1H), 7,70 (d, 1H), 7,49 (d, 1H), 7,17 (t, 1H), 7,05 (d, 2H), 4,10 (q, 2H), 1,36 (t, 3H). [M+H]+: 352,6. |
| 13 | 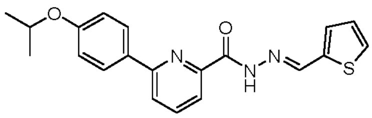 (*E*)-6-(4-isopropoxyphenyl)-*N*'-(thiophen-2-ylmethylene)picolinohydrazide | 400 MHz, DMSO-*d*6 δ 11,86 (s, 1H), 8,94 (s, 1H), 8,13 (d, 1H), 8,05 (t, 1H), 7,96 (d, 1H), 7,70(d, 1H), 7,17 (t, 1H), 7,04 (d, 2H), 4,73 (m, 1H), 1,31 (d, 6H). |
| 14 | 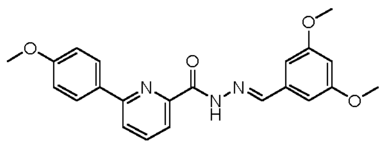 (*E*)-*N'*-(3,5-dimethoxybenzylidene)-6-(4-methoxyphenyl)picolinohydrazide | 400 MHz, DMSO-*d*6 δ 11,90 (s, 1H), 8,66 (s, 1H), 8,32 (d, J = 8,9 Hz, 2H), 8,16 (d, J = 9,2 Hz, 1H), 8,06 (t, J = 7,8 Hz, 1H), 8,00 (d, J = 8,8 Hz, 1H), 7,08 (d, J = 8,9 Hz, 2H), 6,92 (d, J = 2,3 Hz, 2H), 6,60 (t, J = 2,3 Hz, 1H), 3,85 (s, 3H), 3,81 (s, 6H). [M+H]+: 392,5. |
| 15 | 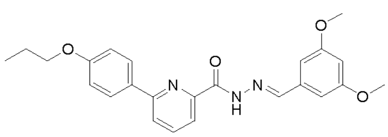 (*E*)-*N'*-(3,5-dimethoxybenzylidene)-6-(4-propoxyphenyl)picolinohydrazide | 400 MHz, DMSO-*d*6 δ 11,90 (s, 1H), 8,66 (s, 1H), 8,31 (d, J = 8,9 Hz, 2H), 8,15 (dd, J = 7,9, 1,2 Hz, 1H), 8,06 (t, J = 7,8 Hz, 1H), 7,99 (t, J = 7,8 Hz, 1H), 7,07 (d, J = 8,9 Hz, 2H), 6,92 (d, J = 2,3 Hz, 2H), 6,60 (t, J = 2,3 Hz, 1H), 4,02 (t, J = 6,6 Hz, 2H), 3,81 (s, 6H), 1,77 (m, 2H), 1,01 (t, J = 7,4 Hz, 3H). [M+H] +: 420,1. |
| 16 | 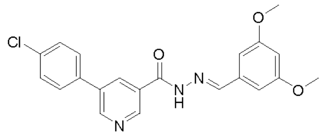 (*E*)-5-(4-chlorophenyl) -*N*'-(3,5-dimethoxybenzylidene)nicotinohydrazide (Compound 16); | 500 MHz, DMSO-*d6* δ 12,09 (s, 1H), 9,10 (d, *J* = 1,9 Hz, 1H), 9,04 (d, *J* = 1,6 Hz, 1H), 8,51 (s, 1H), 8,38 (s, 1H), 7,88 (d, *J* = 8,5 Hz, 2H), 7,62 (d, *J* = 8,5 Hz, 2H), 6,91 (d, *J* = 2,0 Hz, 2H), 6,60 (s, 1H), 3,80 (s, 6H). [M+H]+: 396,0. |

(continued)

| Compound No. | Structure | 1H-NMRI MS m/z [M+H] + |
|---|---|---|
| 17 | 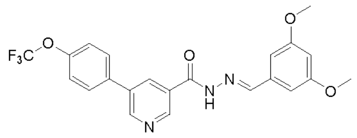 (*E*) -*N'*-(3,5-dimethoxybenzylidene)-5-(4-(trifluoromethoxy)phenyl) nicotinohydrazid e | 500 MHz, DMSO-*d6* 12,01 (s, 1H); 9,11 (d, J = 1,6, 1H); 9,06 (d, J = 1,6, 1H); 8,52 (bs, 1H); 8,38 (s, 1H); 7,97 (d, J = 8,4, 2H); 7,56 (d, J = 8,4, 2H); 6,91 (s, 2H); 6,60 (s, 1H); 3,80 (s, 6H). [M+H]+: 446,6. |
| 18 | 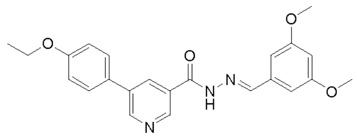 (*E*) -*N'*-(3,5-dimethoxybenzylidene)-5-(4-ethoxyphenyl)nicotinohydrazide (Compound 18); | 500 MHz, DMSO-*d6* $\delta$ 12,01 (s, 1H); 9,04 (d, J = 1,6, 1H); 8,97 (d, J = 1,6, 1H); 8,44 (sl, 1H); 8,38 (s, 1H); 7,77 (d, J = 8,4, 2H); 7,09 (d, J = 8,8, 2H); 6,90 (s, 2H); 6,60 (s, 1H); 4,10 (q, J = 6,8, 2H); 3,80 (s, 6H); 1,36 (t, J = 7,2, 3H). [M+H]+: 406,4. |
| 19 | 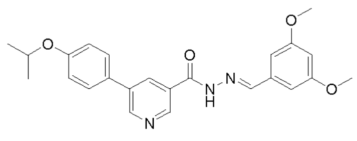 (*E*) -*N'*-(3,5-dimethoxybenzylidene)-5-(4-isopropoxyphenyl)nicotinohydrazide | 500 MHz, DMSO-*d6* $\delta$ 12,06 (1H, s); 9,04 (1H, d, J = 1,6); 8,97 (1H, d, J = 1,6); 8,44 (1H, sl); 8,38 (1H, s); 7,75 (2H, d, J = 8,8); 7,08 (2H, d, J = 8,8); 6,90 (2H, sl); 6,60 (1H, s); 4,71 (1H, sep, J = 6); 3,80 (6H, s); 1,29 (6H, d, J = 6). [M+H]+: 420,5. |
| 20 | 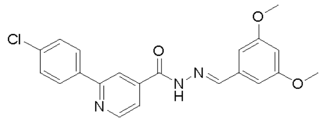 (*E*)-2-(4-chlorophenyl)-*N'*-(3,5-dimethoxybenzylidene)isonicotinohydrazi de | 400 MHz, DMSO-*d6* $\delta$ 12,14 (s, 1H), 8,86 (d, *J* = 5,0 Hz, 1H), 8,41 (s, 1H), 8,37 (s, 1H), 8,20 (d, *J* = 8,6 Hz, 2H), 7,82 - 7,77 (m, 1H), 7,61 (d, *J* = 8,6 Hz, 2H), 6,91 (d, *J* = 2,1 Hz, 2H), 6,61 (t, *J* = 2,1 Hz, 1H), 3,80 (s, 6H). [M+H]+: 396,6. |
| 21 | 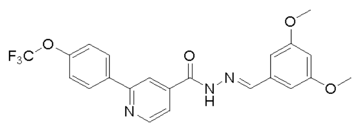 (*E*) -*N'*-(3,5-dimethoxybenzylidene)-2-(4-(trifluoromethoxy)phenyl) isonicotinohydra zide | 400 MHz, DMSO-*d6* $\delta$ 12,11 (s, 1H), 8,79 (d, *J* = 5,0 Hz, 1H), 8,41 (s, 1H), 8,26 (s, 1H), 8,10 (d, *J* = 8,8 Hz, 2H), 7,71 - 7,66 (m, 1H), 7,06 (d, *J* = 8,8 Hz, 2H), 6,91 (d, *J* = 2,2 Hz, 2H), 6,61 (t, *J* = 2,1 Hz, 1H), 4,71 (h, *J* = 6,0 Hz, 1H), 3,80 (s, 6H), 1,30 (d, *J* = 6,0 Hz, 6H). [M+H]+: 445,8. |
| 22 | 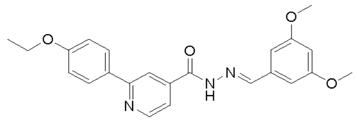 (*E*)-*N'*-(3,5-dimethoxybenzylidene)-2-(4-ethoxyphenyl)isonicotinohydrazide | 400 MHz, DMSO-*d6* $\delta$ 12,11 (s, 1H), 8,80 (d, *J* = 4,9 Hz, 1H), 8,41 (s, 1H), 8,27 (s, 1H), 8,11 (d, *J* = 8,8 Hz, 2H), 7,71 - 7,66 (m, 1H), 7,07 (d, *J* = 8,8 Hz, 2H), 6,91 (d, *J* = 2,1 Hz, 2H), 6,61 (t, *J* = 2,1 Hz, 1H), 4,11 (q, *J* = 6,9 Hz, 2H), 3,80 (s, 6H), 1,36 (t, *J* = 7,0 Hz, 3H). [M+H]+: 406,3. |
| 23 | 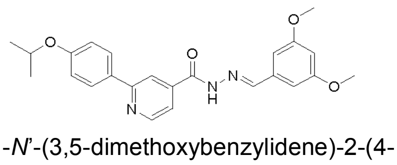 (*E*) -*N'*-(3,5-dimethoxybenzylidene)-2-(4-isopropoxyphenyl)isonicotinohydrazide | 400 MHz, DMSO-*d6* $\delta$ 12,11 (s, 1H), 8,79 (d, *J* = 5,0 Hz, 1H), 8,41 (s, 1H), 8,26 (s, 1H), 8,10 (d, *J* = 8,8 Hz, 2H), 7,71 - 7,66 (m, 1H), 7,06 (d, *J* = 8,8 Hz, 2H), 6,91 (d, *J* = 2,2 Hz, 2H), 6,61 (t, *J* = 2,1 Hz, 1H), 4,71 (h, *J* = 6,0 Hz, 1H), 3,80 (s, 6H), 1,30 (d, *J* = 6,0 Hz, 6H). [M+H]+: 420,0. |

(continued)

| Compound No. | Structure | 1H-NMRI MS m/z [M+H] + |
|---|---|---|
| 24 | 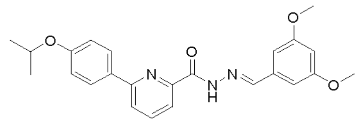 (*E*) -*N*'-(3,5-dimethoxybenzylidene)-6-(4-isopropoxyphenyl)picolinohydrazide | 400 MHz, DMSO-*d6* δ 11,91 (s, 1H), 8,65 (s, 1H), 8,30 (d, *J* = 8,8 Hz, 2H), 8,16 (d, *J* = 8,0 Hz, 1H), 8,06 (t, *J* = 7,8 Hz, 1H), 7,99 (d, *J* = 7,4 Hz, 1H), 7,06 (d, *J* = 8,8 Hz, 2H), 6,92 (d, *J* = 2,1 Hz, 2H), 6,60 (s, 1H), 4,74 (m, 1H), 3,81 (s, 6H), 1,31 (d, *J* = 6,0 Hz, 6H). |
| 25 | 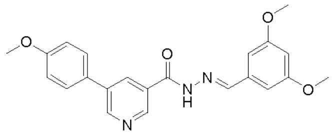 (*E*)-*N*'-(3,5-dimethoxybenzylidene)-5-(4-methoxyphenyl)nicotinohydrazide | 400 MHz, DMSO-*d6* δ 12,18 (s, 1H), 9,08 (d, *J* = 2,0 Hz, 1H), 9,00 (d, *J* = 1,9 Hz, 1H), 8,53 (s, 1H), 8,41 (s, 1H), 7,81 (H-11, d, *J* = 8,7 Hz, 2H), 7,12 (H-12, d, *J* = 8,8 Hz, 2H), 6,91 (d, *J* = 2,2 Hz, 2H), 6,60 (t, *J* = 2,3 Hz, 1H), 3,83 (s, 3H), 3,80 (H-29, s, 6H). [M+H]+: 392,5. |
| 26 | 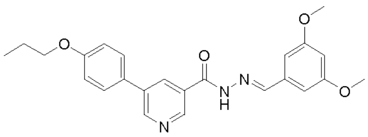 (*E*)-*N*'-(3,5-dimethoxybenzylidene)-5-(4-propoxyphenyl)nicotinohydrazide | 400 MHz, DMSO-*d6* δ 12,10 (s, 1H), 9,05 (d, *J* = 2,2 Hz, 1H), 8,97 (d, *J* = 1,9 Hz, 1H), 8,46 (t, *J* = 2,1 Hz, 1H), 8,38 (s, 1H), 7,78 (d, *J* = 8,8 Hz, 2H), 7,10 (d, *J* = 8,8 Hz, 2H), 6,91 (d, *J* = 2,2 Hz, 2H), 6,60 (t, *J* = 2,3 Hz, 1H), 4,00 (t, *J* = 6,5 Hz, 2H), 3,80 (s, 6H), 1,76 (q, *J* = 6,9 Hz, 2H), 1,00 (t, *J* = 7,4 Hz, 3H). [M+H]+: 420,4. |
| 27 | 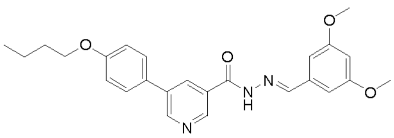 (*E*)-5-(4-butoxyphenyl)-*N'*-(3,5-dimethoxybenzylidene)nicotinohydrazide | 400 MHz, DMSO-*d6* δ 12,13 (s, 1H), 9,06 (d, *J* = 2,2 Hz, 1H), 8,98 (d, *J* = 2,0 Hz, 1H), 8,49 (t, *J* = 2,0 Hz, 1H), 8,39 (s, 1H), 7,78 (d, *J* = 8,8 Hz, 2H), 7,10 (d, *J* = 8,8 Hz, 2H), 6,91 (d, *J* = 2,3 Hz, 2H), 6,60 (t, *J* = 2,2 Hz, 1H), 4,04 (t, *J* = 6,5 Hz, 2H), 3,80 (s, 6H), 1,76 - 1,69 (m, 2H), 1,46 (m, 2H), 0,95 (t, *J* = 7,4 Hz, 3H). [M+H]+: 434,2. |
| 28 | 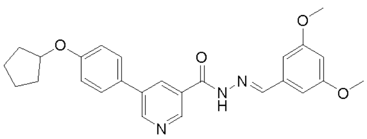 (*E*)-5-(4-(cyclopentyloxy)phenyl)-*N'*-(3,5-dimethoxybenzylidene)nicotinohydrazide | 400 MHz, DMSO-*d6* δ 12,07 (s, 1H), 9,04 (d, *J* = 1,9 Hz, 1H), 8,96 (d, *J* = 1,6 Hz, 1H), 8,44 (sl, 1H), 8,38 (s, 1H), 7,75 (d, *J* = 8,6 Hz, 2H), 7,06 (d, *J* = 8,7 Hz, 2H), 6,91 (d, *J* = 2,1 Hz, 2H), 6,60 (t, *J* = 1,9 Hz, 1H), 4,89 - 4,92 (m, 1H), 3,80 (6H), 2,00 - 1,89 (m, 2H), 1,79 - 1,69 (m, 4H), 1,63 - 1,56 (m, 2H). [M+H]+: 446,2. |
| 29 | 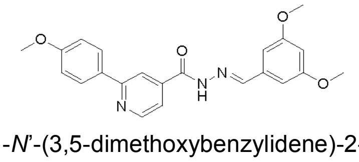 (*E*) -*N*'-(3,5-dimethoxybenzylidene)-2-(4-methoxyphenyl)isonicotinohydrazide | 400 MHz, DMSO-*d6* δ 12,10 (s, 1H), 8,80 (d, *J* = 5,0 Hz, 1H), 8,41 (s, 1H), 8,27 (s, 1H), 8,11 (d, *J* = 8 Hz, 2H), 7,69 (dd, *J* = 5,0, 1,5 Hz, 1H), 7,09 (d, *J* = 8 Hz, 2H), 6,91 (sinais sobrepostos, 2H), 6,61 (t, *J* = 2,3 Hz, 1H), 3,84 (s, 3H), 3,81 (s, 6H). [M+H]+: 392,0. |
| 30 | 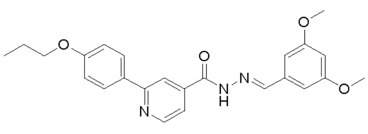 (*E*) -*N*'-(3,5-dimethoxybenzylidene)-2-(4-propoxyphenyl)isonicotinohydrazide (Compound 30); | 400 MHz, DMSO-*d6* δ 12,10 (s, 1H), 8,79 (d, *J* = 4,9 Hz, 1H), 8,42 (s, 1H), 8,27 (s, 1H), 8,11 (d, *J* = 8,6 Hz, 2H), 7,68 (d, *J* = 4,9 Hz, 1H), 7,08 (d, *J* = 8,6 Hz, 2H), 6,91 (s, 2H), 6,61 (s, 1H), 4,00 (t, *J* = 6,5 Hz, 2H), 3,80 (s, 6H), 1,77 (dt, *J* = 13,9, 7,0 Hz, 2H), 1,00 (t, *J* = 7,4 Hz, 3H). [M+H]+: 420,3. |

(continued)

| Compound No. | Structure | 1H-NMRI MS m/z [M+H] + |
|---|---|---|
| 31 | 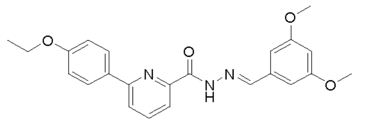<br>(*E*) -*N*'-(3,5-dimethoxybenzylidene)-6-(4-ethoxyphenyl)picolinohydrazide (Compound 31); | 400 MHz, DMSO-*d6* $\delta$ 11,91 (s, 1H), 8,65 (s, 1H), 8,32 (d, *J* = 8,9 Hz, 2H), 8,16 (dd, *J* = 8,0, 0,9 Hz, 1H), 8,06 (t, *J* = 7,8 Hz, 1H), 7,99 (dd, *J* = 7,6, 0,9 Hz, 1H), 7,07 (d, *J* = 8,9 Hz, 2H), 6,92 (sinais sobrepostos, 2H), 6,60 (t, *J* = 2,3 Hz, 1H), 4,12 (q, *J* = 6,9 Hz, 2H), 3,81 (s, 6H), 1,35 (s, 3H). |
| 32 | 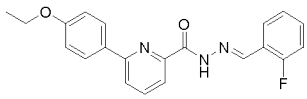<br>(*E*)-6-(4-ethoxyphenyl) -*N*'-(2-fluorobenzylidene)picolinohydrazide | 400 MHz, DMSO-*d6* $\delta$ 12,09 (1H), 9,00 (s, 1H), 8,33 (d, *J* = 8,8 Hz, 2H), 8,16 (d, *J* = 7,9 Hz, 1H), 8,06 (t, *J* = 7,8 Hz, 1H), 8,01 (m, 2H), 7,52 (m, 1H), 7,33 (t, *J* = 8,6 Hz, 2H), 7,08 (d, *J* = 8,8 Hz, 2H), 4,12 (q, *J* = 7,0 Hz, 2H), 1,37 (t, *J* = 6,9 Hz, 3H). [M+H]+: 364,4. |
| 33 | 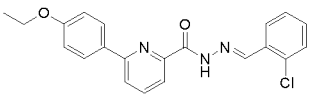<br>(*E*) -*N*'-(2-chlorobenzylidene)-6-(4-ethoxyphenyl)picolinohydrazide | 400 MHz, DMSO-*d6* $\delta$ 12,23 (s, 1H), 9,12 (s, 1H), 8,29 (d, *J* = 8,2 Hz, 2H), 8,13 (d, *J* = 7,9 Hz, 1H), 8,09 - 8,00 (m, 2H), 7,99 (d, *J* = 5 Hz), 7,52 (d, *J* = 7,5 Hz, 1H), 7,48 - 7,40 (m, 2H), 7,05 (d, *J* = 8,2 Hz, 2H), 4,09 (q, *J* = 6,8 Hz, 2H), 1,34 (t, *J* = 6,8 Hz, 3H). [M+H]+: 380,4. |
| 34 | 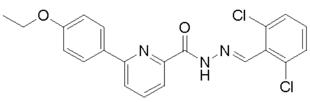<br>(*E*) -*N'*-(2,6-dichlorobenzylidene)-6-(4-ethoxyphenyl)picolinohydrazide | 400 MHz, DMSO-*d6* $\delta$ 12,26 (s, 1H), 8,92 (s, 1H), 8,32 (d, *J* = 8,8 Hz, 2H), 8,17 (d, *J* = 8,0 Hz, 1H), 8,07 (t, *J* = 7,8 Hz, 1H), 7,99 (d, *J* = 7,6 Hz, 1H), 7,60 (d, *J* = 8,2 Hz, 2H), 7,47 (t, *J* = 8,1 Hz, 1H), 7,08 (d, *J* = 8,8 Hz, 2H), 4,12 (q, *J* = Hz, 2H), 1,37 (t, *J* = 6,9 Hz, 3H). [M+H]+: 414,1. |
| 35 | 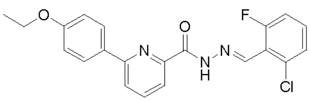<br>(*E*)-*N'*-(2-chloro-6-fluorobenzylidene)-6-(4-ethoxyphenyl)picolinohydrazide | 400 MHz, DMSO-*d6* $\delta$ 12,21 (s, 1H), 8,96 (s, 1H), 8,32 (d, *J* = 8,8 Hz, 2H), 8,17 (d, *J* = 8,0 Hz, 1H), 8,07 (t, *J* = 7,8 Hz, 1H), 7,99 (d, *J* = 7,6 Hz, 1H), 7,55 - 7,47 (m, 1H), 7,45 (d, *J* = 7,9 Hz, 1H), 7,40 - 7,33 (m, 1H), 7,08 (d, *J* = 8,8 Hz, 2H), 4,12 (q, *J* = 6,9 Hz, 2H), 1,37 (t, *J* = 7,0 Hz, 3H). [M+H]+: 398,0. |
| 36 | 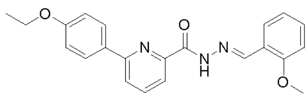<br>(*E*)-6-(4-ethoxyphenyl) -*N'*-(2-methoxybenzylidene)picolinohydrazide | 400 MHz, DMSO-*d6* $\delta$ 12,00 (s, 1H), 9,04 (s, 1H), 8,33 (d, *J* = 8,8 Hz, 2H), 8,14 (d, *J* = 7,9 Hz, 1H), 8,05 (t, *J* = 7,7 Hz, 1H), 7,98 (d, *J* = 7,5 Hz, 1H), 7,92 (dd, *J* = 7,7, 1,6 Hz, 1H) 7,49 - 7,40 (dt, *J* = 12 Hz, 4 Hz, 1H), 7,13 (d, *J* = 8,3 Hz, 1H), 7,06 (dd, *J* = 14,8, 8,2 Hz, 3H), 4,12 (q, *J* = 7,0 Hz, 2H), 3,89 (s, 3H), 1,37 (t, *J* = 7,0 Hz, 3H). [M+H]+: 376,3. |
| 37 | 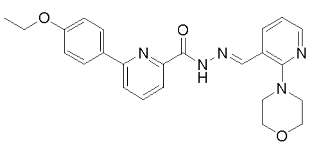<br>(*E*)-6-(4-ethoxyphenyl) -*N'*-((2-morpholinopyridin-3-yl)methylene) picolinohydrazide | 400 MHz, DMSO-*d6* $\delta$ 12,09 (s, 1H), 8,77 (s, 1H), 8,33 (dd, *J* = 4,7, 1,9 Hz, 1H), 8,29 (d, *J* = 8,8 Hz, 2H), 8,19 - 8,14 (m, sinais solapados, 2H), 8,06 (t, *J* = 7,8 Hz, 1H), 7,98 (d, *J* = 7,1 Hz, 1H), 7,13 (dd, *J* = 7,6, 4,8 Hz, 1H), 7,08 (d, *J* 8,8 Hz, 2H), 4,12 (q, *J* = 7,0 Hz, 2H), 3,85 - 3,76 (m, 4H), 3,22 - 3,14 (m, 4H), 1,37 (t, *J* = 7,0 Hz, 3H). [M+H]+: 431,9. |

(continued)

| Compound No. | Structure | 1H-NMRI MS m/z [M+H] + |
|---|---|---|
| 38 | 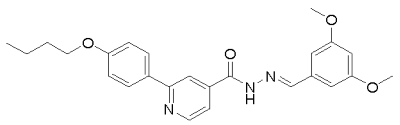 (*E*)-2-(4-butoxyphenyl)-*N'*-(3,5-dimethoxybenzylidene)isonicotinohydrazi de | 400 MHz, DMSO-*d6* $\delta$ 12,10 (s, 1H), 8,79 (d, *J* = 5,4 Hz, 1H), 8,41 (s, 1H), 8,27 (s, 1H), 8,11 (d, *J* = 6,9 Hz, 2H), 7,68 (dd, *J* = 5,0, 1,5 Hz, 1H), 7,08 (d, *J* = 8,9 Hz, 2H), 6,91(sinais sobrepostos, 2H), 6,61 (t, *J* = 2,3 Hz, 1H), 4,05 (t, *J* = 6,5 Hz, 2H), 3,81 (s, 6H), 1,73 (dt, *J* = 14,3, 6,5 Hz, 2H), 1,47 (dt, *J* = 14,9, 7,4 Hz, 2H), 0,95 (t, *J* = 7,4 Hz, 3H). [M+H]+: 434,2. |
| 39 | 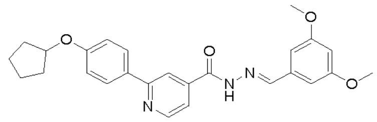 (*E*)-2-(4-(cyclopentyloxy)phenyl) -*N'*-(3,5-dimethoxybenzylidene)isonicotinohydrazi de | 400 MHz, DMSO-*d6* $\delta$ 12,10 (s, 1H), 8,79 (d, *J* = 5,0 Hz, 1H), 8,41 (s, 1H), 8,26 (s, 1H), 8,09 (d, *J* = 8,8 Hz, 2H), 7,68 (dd, *J* = 5,0, 1,3 Hz, 1H), 7,05 (d, *J* = 8,8 Hz, 2H), 6,91 (sinais sobrepostos, 2H), 6,61 (t, *J* = 2,2 Hz, 1H), 4,91 (s, 1H), 3,81 (s, 6H), 1,95 (m, 2H), 1,74 (m, 4H), 1,61 (m, 2H). [M+H]+: 445,7. |
| 40 | 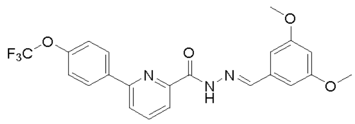 (*E*) -*N'*-(3,5-dimethoxybenzylidene)-6-(4-(trifluoromethoxy)phenyl) picolinohydrazid e | 400 MHz, DMSO-*d6* $\delta$ 11,95 (s, 1H), 8,65 (s, 1H), 8,49 (d, *J* = 8,9 Hz, 2H), 8,27 (dd, *J* = 7,9, 1,0 Hz, 1H), 8,16 (t, *J* = 7,8 Hz, 1H), 8,10 (dd, *J* = 7,7,1,0 Hz, 1H), 7,53 (d, *J* = 8,1 Hz, 2H), 6,92 (overlapping signs, 2H), 6,60 (t, *J* = 2,3 Hz, 1H), 3,81 (s, 6H). [M+H]+: 445,7. |
| 41 | 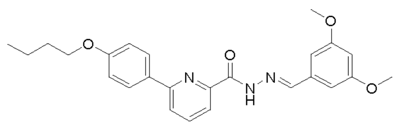 (*E*)-6-(4-butoxyphenyl) -*N'*-(3,5-dimethoxybenzylidene)picolinohydrazide | 400 MHz, DMSO-*d6* $\delta$ 11,90 (s, 1H), 8,66 (s, 1H), 8,30 (d, *J* = 8,9 Hz, 2H), 8,14 (d, *J* = 7,9 Hz, 1H), 8,05 (H-3, t, *J* = 7,8 Hz, 1H), 7,99 (d, *J* = 8,3 Hz, 1H), 7,06 (d, *J* = 8,9 Hz, 2H), 6,92 (sinais sobrepostos, 2H), 6,60 (t, *J* = 2,3 Hz, 1H), 4,05 (t, *J* = 6,5 Hz, 2H), 3,79 (s, 6H), 1,73 (dt, *J* = 14,3, 6,5 Hz, 2H), 1,46 (h, *J* = 7,4 Hz, 2H), 0,95 (t, *J* = 7,4 Hz, 3H). [M+H]+: 434,3. |
| 42 | 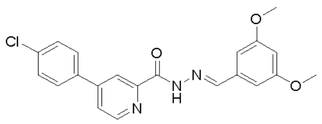 (*E*)-2-(4-chlorophenyl)-*N'*-(3,5-dimethoxybenzylidene)isonicotinohydrazi de | 400 MHz, DMSO-*d*6 $\delta$ 12,19 (s, 1H), 8,77 (d, *J* = 5,3 Hz, 1H), 8,57 (s, 1H), 8,34 (s, 1H), 7,99 (dd, *J* = 1,8, 5,1 Hz, 1H), 7,92 (d, *J* = 8,6 Hz, 2H), 7,62 (d, *J* = 8,6 Hz, 2H), 6,85 (d, *J* = 2,0 Hz, 2H), 6,58 (s, 1H), 3,79 (s, 6H). [M+H]+: 396,1. |
| 43 | 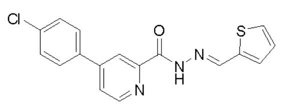 (*E*)-6-(4-chlorophenyl) -*N'*-(thiophen-2-ylmethylene)picolinohydrazide | 400 MHz, DMSO-*d6* $\delta$ 12,20 (s, 1H), 8,84 (s, 1H), 8,76 (d, *J* = 5,1 Hz, 1H), 8,33 (s, 1H), 7,98 (dd, *J* = 1,7, 5,0 Hz, 1H), 7,92 (d, *J* = 8,4 Hz, 2H), 7,68 (d, *J* = 4,9 Hz, 1H), 7,61 (d, *J* = 8,6 Hz, 2H), 7,42 (d, *J* = 3,1 Hz, 1H), 7,19 - 7,08 (m, 1H). [M+H]+: 342,0. |
| 44 | 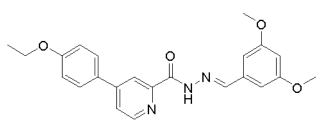 (*E*) -*N'*-(3,5-dimethoxybenzylidene)-6-(4-ethoxyphenyl)picolinohydrazide | 400 MHz, DMSO-*d6* $\delta$ 12,18 (s, 1H), 8,71 (d, *J* = 5,3 Hz, 1H), 8,59 (s, 1H), 8,32 (d, *J* = 1,2 Hz, 1H), 7,95 (dd, *J* = 1,9, 5,2 Hz, 1H), 7,86 (d, *J* = 8,8 Hz, 2H), 7,11 (d, *J* = 8,8 Hz, 2H), 6,87 (d, *J* = 2,2 Hz, 2H), 6,60 (t, *J* = 2,3 Hz, 1H), 4,12 (q, *J* = 7,0 Hz, 2H), 3,81 (s, 6H), 1,37 (t, *J* = 7,0 Hz, 3H). [M+H]+: 406,1. |

(continued)

| Compound No. | Structure | 1H-NMRI MS m/z [M+H] + |
|---|---|---|
| 45 | 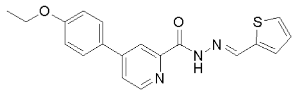 (*E*)-6-(4-ethoxyphenyl) -*N*'-(thiophen-2-ylmethylene)picolinohydrazide | 400 MHz, DMSO-*d6* δ 12,18 (s, 1H), 8,85 (s, 1H), 8,70 (d, *J* = 5,3 Hz, 1H), 8,30 (d, *J* = 1,3 Hz, 1H), 7,94 (dd, *J* = 2,0, 5,1 Hz, 1H), 7,85 (d, *J* = 8,8 Hz, 2H), 7,69 (d, *J* = 5,0 Hz, 1H), 7,48 - 7,41 (m, 1H), 7,21 - 7,06 (m, 3H), 4,11 (q, *J* = 7,0 Hz, 2H), 1,36 (t, *J* = 7,0 Hz, 3H). [M+H]+: 352,0. |
| 46 | 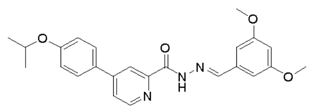 (*E*) -*N*'-(3,5-dimethoxybenzylidene)-6-(4-isopropoxyphenyl)picolinohydrazide | 400 MHz, DMSO-*d6* δ 2,19 (s, 1H), 8,71 (d, *J* = 5,3 Hz, 1H), 8,58 (s, 1H), 8,31 (s, 1H), 7,95 (dd, *J* = 1,8, 5,3 Hz, 1H), 7,84 (d, *J* = 8,8 Hz, 2H), 7,09 (d, *J* = 8,8 Hz, 2H), 6,86 (d, *J* = 2,0 Hz, 2H), 6,60 - 6,58 (m, 1H), 4,73 (td, *J* = 6,0, 12,1 Hz, 1H), 3,81 (s, 6H), 1,31 (d, *J* = 6,0 Hz, 6H). [M+H]+: 420,1. |
| 47 | 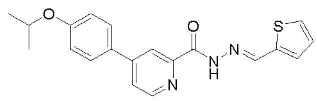 (*E*)-4-(4-isopropoxyphenyl)-*N*'-(thiophen-2-ylmethylene)picolinohydrazide | 400 MHz, DMSO-*d*6 δ 12,20 (s, 1H), 8,85 (s, 1H), 8,70 (d, *J* = 5,1 Hz, 1H), 8,30 (d, *J* = 1,3 Hz, 1H), 7,94 (dd, *J* = 1,9, 5,2 Hz, 1H), 7,84 (d, *J* = 8,8 Hz, 2H), 7,70 (d, *J* = 5,1 Hz, 1H), 7,44 (d, *J* = 2,9 Hz, 1H), 7,16 (dd, *J* = 3,7, 4,9 Hz, 1H), 7,09 (d, *J* = 8,6 Hz, 2H), 4,79 - 4,66 (m, 1H), 1,31 (d, *J* = 6,0 Hz, 6H). [M+H]+: 366,1. |
| 48 | 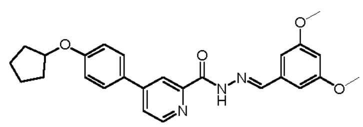 (*E*)-4-(4-(cyclopentyloxy)phenyl) -*N*'-(3,5-dimethoxybenzylidene)picolinohydrazide | 400 MHz, CDCl*3* δ 11,09 - 11,01 (m, 1H), 8,61 - 8,49 (m, 2H), 8,31 - 8,22 (m, 1H), 7,72 - 7,62 (m, 3H), 6,98 (s, 4H), 6,57 - 6,48 (m, 1H), 4,87 - 4,80 (m, 1H), 3,85 (s, 6H), 2,02 - 1,75 (m, 6H), 1,72 - 1,61 (m, 2H). [M+H] +: 446,1. |
| 49 | 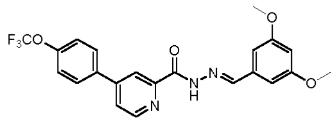 (*E*) -*N*'-(3,5-dimethoxybenzylidene)-6-(4-(trifluoromethoxy)phenyl) picolinohydrazid e | 400 MHz, CDCl*3* δ 10,95 (s, 1H), 8,57 (d, *J* = 5,1 Hz, 1H), 8,46 (d, *J* = 1,2 Hz, 1H), 8,19 (s, 1H), 7,70 (d, *J* = 7,4 Hz, 2H), 7,61 (dd, *J* = 1,9, 5,1 Hz, 1H), 7,30 (d, *J* = 8,1 Hz, 2H), 7,19 (s, 1H), 6,91 (d, *J* = 2,3 Hz, 2H), 6,46 (t, *J* = 2,3 Hz, 1H), 3,78 (s, 6H). [M+H]+: 446,1. |
| 50 | 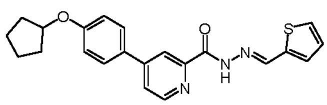 (*E*)-4-(4-(cyclopentyloxy) phenyl)-*N*'-(thiophen-2-ylmethylene) picolinohydrazide | 400 MHz, CDCl*3* δ 11,02 - 10,94 (m, 1H), 8,76 - 8,69 (m, 1H), 8,57 - 8,53 (m, 1H), 8,50 - 8,47 (m, 1H), 7,72 - 7,62 (m, 3H), 7,47 - 7,41 (m, 1H), 7,38 - 7,35 (m, 1H), 7,11 - 7,05 (m, 1H), 7,02 - 6,96 (m, 2H), 4,88 - 4,79 (m, 1H), 2,02 - 1,76 (m, 6H), 1,72 - 1,61 (m, 2H). [M+H]+: 392,1. |
| 51 | 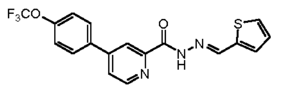 (*E*) -*N*'-(thiophen-2-ylmethylene)-4-(4-(trifluoromethoxy)phenyl) picolinohydrazid e | 400 MHz, CDCl*3* δ 10,88 (s, 1H), 8,65 (s, 1H), 8,56 (d, *J* = 5,1 Hz, 1H), 8,43 (d, *J* = 1,2 Hz, 1H), 7,69 (d, *J* = 7,4 Hz, 2H), 7,59 (dd, *J* = 1,8, 5,0 Hz, 1H), 7,38 (d, *J* = 5,0 Hz, 1H), 7,33 - 7,28 (m, 3H), 7,19 (s, 1H), 7,02 (dd, *J* = 3,7, 5,0 Hz, 1H). [M+H]+: 392,1. |

(continued)

| Compound No. | Structure | 1H-NMRI MS m/z [M+H] + |
|---|---|---|
| 52 | 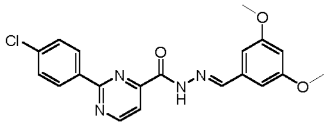 (*E*)-2-(4-chlorophenyl) -*N*'-(3,5-dimethoxybenzylidene)pyrimidine-4-carbohydrazide | 400 MHz, DMSO-*d6* δ 3,74 - 3,85 (m, 6 H) 6,61 (t, J = 2,20 Hz, 1 H) 6,92 (d, J = 2,21 Hz, 2 H) 7,64 (d, J = 8,60 Hz, 2 H) 8,00 (d, J = 4,85 Hz, 1 H) 8,63 - 8,73 (m, 3 H) 9,18 (d, J = 4,85 Hz, 1 H) 12,20 (s, 1 H). [M+H]+: 397,2. |
| 53 | 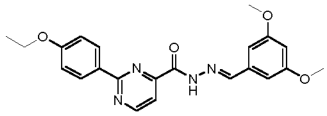 (*E*) -*N*'-(3,5-dimethoxybenzylidene)-2-(4-ethoxyphenyl)pyrimidine-4-carbohydrazide | 400 MHz, CDCl*3* δ 10,92 (s, 1H), 9,02 (d, J = 4,8 Hz, 1H), 8,44 - 8,39 (m, 3H), 8,00 (d, J = 4,8 Hz, 1H), 7,05 - 6,98 (m, 4H), 6,55 (s, 1H), 4,14 (q, J = 6,7 Hz, 2H), 3,85 (s, 6H), 1,47 (t, J = 6,8 Hz, 3H). [M+H]+: 407,1. |
| 54 | 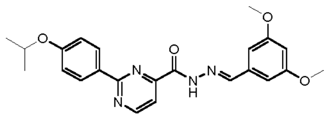 (*E*) -*N*'-(3,5-dimethoxybenzylidene)-2-(4-isopropoxyphenyl)pyrimidine-4-carbohydrazide | 400 MHz, DMSO-*d6* δ 12,14 (s, 1H), 12,18 - 12,10 (m, 1H), 9,11 (d, J = 4,9 Hz, 1H), 8,70 (s, 1H), 8,61 (d, J = 8,8 Hz, 2H), 7,90 (d, J = 4,9 Hz, 1H), 7,09 (d, J = 8,9 Hz, 2H), 6,94 (d, J = 2,3 Hz, 2H), 6,63 (s, 1H), 4,84 - 4,73 (m, 1H), 3,82 (s, 6H), 1,33 (d, J = 6,0 Hz, 6H). [M+H]+: 421,1. |
| 55 | 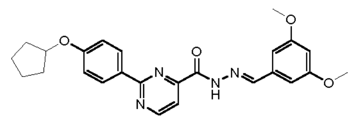 (*E*)-2-(4-(cyclopentyloxy)phenyl) -*N*'-(3,5-dimethoxybenzylidene)pyrimidine-4-carbohydrazide | 400 MHz, DMSO-*d6* δ 12,14 (s, 1H), 9,11 (d, J = 4,9 Hz, 1H), 8,70 (s, 1H), 8,61 (d, J = 8,8 Hz, 2H), 7,90 (d, J = 4,9 Hz, 1H), 7,07 (d, J = 8,8 Hz, 2H), 6,94 (d, J = 2,1 Hz, 2H), 6,63 (s, 1H), 5,01 - 4,91 (m, 1H), 3,82 (s, 6H), 1,99 (br t, J = 5,6 Hz, 2H), 1,81 - 1,68 (m, 4H), 1,62 (br s, 2H). [M+H]+: 447,1. |
| 56 | 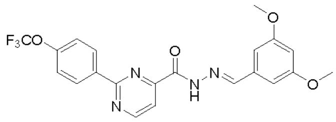 (*E*) -*N*'-(3,5-dimethoxybenzylidene) -2-(4-(trifluoromethoxy)phenyl)pyrimidine-4-carbohydrazide | 400 MHz, DMSO-*d6* δ 12,21 (s, 1H), 9,22 (d, *J* = 4,9 Hz, 1H), 8,80 (d, *J* = 8,0 Hz, 2H), 8,70 (s, 1H), 8,04 (d, *J* = 5,0 Hz, 1H), 7,58 (d, *J* = 8,1 Hz, 2H), 6,94 (d, *J* = 2,2 Hz, 2H), 6,63 (t, *J* = 2,3 Hz, 1H), 3,82 (s, 6H). [M+H]+: 447,1. |
| 57 | 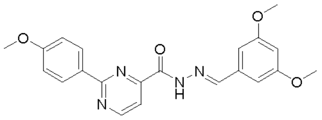 (*E*) -*N*'-(3,5-dimethoxybenzylidene)-2-(4-methoxyphenyl)pyrimidine-4-carbohydrazide | 400 MHz, DMSO-*d6* δ 10,97 - 10,86 (m, 1H), 9,06 - 8,95 (m, 1H), 8,47 - 8,37 (m, 3H), 8,04 - 7,94 (m, 1H), 7,08 - 7,02 (m, 2H), 7,00 - 6,96 (m, 2H), 6,59 - 6,49 (m, 1H), 3,91 (s, 3H), 3,87 - 3,82 (m, 6H). [M+H]+: 393,6. |
| 58 | 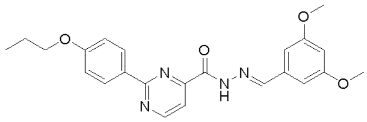 (E) -N '-(3,5-dimethoxybenzylidene)-2-(4-isopropoxyphenyl)pyrimidine-4-carbohydrazide | 400 MHz, DMSO-*d6* δ 10,95 (s, 1H), 9,04 (d, J = 4,9 Hz, 1H), 8,48 - 8,41 (m, 3H), 8,03 (d, J = 4,8 Hz, 1H), 7,09 - 6,99 (m, 4H), 6,58 (t, J = 2,1 Hz, 1H), 4,05 (t, J = 6,5 Hz, 2H), 3,88 (s, 6H), 1,89 (sxt, J = 7,1 Hz, 2H), 1,11 (t, J = 7,5 Hz, 3H). [M+H]+: 421,1. |

(continued)

| Compound No. | Structure | 1H-NMRI MS m/z [M+H] + |
|---|---|---|
| 59 | 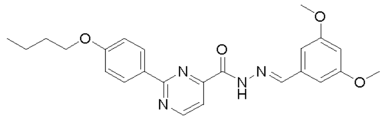 (*E*)-2-(4-butoxyphenyl) -*N*'-(3,5-dimethoxybenzylidene)pyrimidine-4-carbohydrazide | 400 MHz, DMSO-*d6* δ 12,14 (s, 1H), 9,12 (d, *J* = 5,0 Hz, 1H), 8,70 (s, 1H), 8,65 - 8,59 (m, *J* = 8,9 Hz, 2H), 7,91 (d, *J* = 5,0 Hz, 1H), 7,14 - 7,07 (m, *J* = 8,9 Hz, 2H), 6,94 (d, *J* = 2,3 Hz, 2H), 6,63 (t, *J* = 2,3 Hz, 1H), 4,10 (t, *J* = 6,5 Hz, 2H), 3,82 (s, 6H), 1,80 - 1,71 (m, 2H), 1,48 (sxt, *J* = 7,5 Hz, 2H), 0,97 (t, *J* = 7,4 Hz, 3H). [M+H]+: 435,1. |
| 60 | 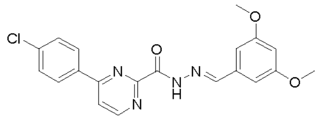 (*E*)-4-(4-chlorophenyl) -*N*'-(3,5-dimethoxybenzylidene)pyrimidine-2-carbohydrazide | 400 MHz, DMSO-*d6* δ 11,90 (s, 1H), 9,03 (br d, *J* = 4,6 Hz, 1H), 8,58 (br s, 1H), 8,36 (br d, *J* = 7,9 Hz, 2H), 8,21 (br d, *J* = 4,2 Hz, 1H), 7,63 (br d, *J* = 8,4 Hz, 2H), 6,90 (br s, 2H), 6,58 (br s, 1H), 3,81 (br s, 6H). [M+H]+: 397,1. |
| 61 | 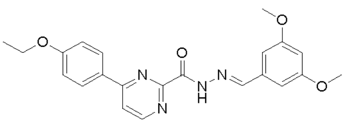 (*E*) -*N*'-(3,5-dimethoxybenzylidene)-4-(4-ethoxyphenyl)pyrimidine-2-carbohydrazide | 400 MHz, DMSO-*d6* δ 11,88 (br s, 1H), 8,91 (br d, *J* = 4,9 Hz, 1H), 8,57 (br s, 1H), 8,30 (br d, *J* = 8,2 Hz, 2H), 8,11 (br d, *J* = 4,9 Hz, 1H), 7,10 (br d, *J* = 7,9 Hz, 2H), 6,90 (brs, 2H), 6,58 (brs, 1H), 4,16 (brd, *J* = 6,0 Hz, 2H), 3,81 (br s, 6H), 1,37 (br t, *J* = 6,5 Hz, 3H). [M+H]+: 407,1. |
| 62 | 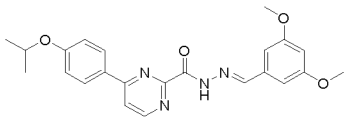 (*E*) -*N*'-(3,5-dimethoxybenzylidene)-4-(4-isopropoxyphenyl)pyrimidine-2-carbohydrazide | 400 MHz, DMSO-*d6* δ 11,85 (s, 1H), 8,91 (br d, *J* = 5,3 Hz, 1H), 8,57 (br s, 1H), 8,28 (br d, *J* = 7,7 Hz, 2H), 8,09 (br d, *J* = 4,2 Hz, 1H), 7,08 (br d, *J* = 8,4 Hz, 2H), 6,90 (br s, 2H), 6,58 (br s, 1H), 4,74 (td, *J* = 6,0, 11,8 Hz, 1H), 3,81 (br s, 6H), 1,32 (d, *J* = 6,0 Hz, 6H). [M+H]+: 421,2. |
| 63 | 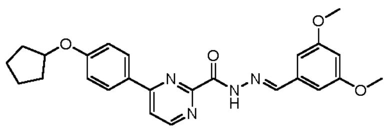 (*E*)-4-(4-(cyclopentyloxy)phenyl) -*N*'-(3,5-dimethoxybenzylidene)pyrimidine-2-carbohydrazide | 400 MHz, DMSO-*d6* δ 12,15 (s, 1H), 8,96 (d, J = 5,5 Hz, 1H), 8,58 (s, 1H), 8,36 (d, J = 8,9 Hz, 2H), 8,19 (d, J = 5,5 Hz, 1H), 7,10 (d, J = 8,9 Hz, 2H), 6,91 (d, J = 2,2 Hz, 2H), 6,61 (t, J = 2,3 Hz, 1H), 4,97 (br t, J = 5,9 Hz, 1H), 3,82 (s, 6H), 2,03 - 1,93 (m, 2H), 1,79 - 1,69 (m, 4H), 1,67 - 1,57 (m, 2H). [M+H]+: 447,1. |
| 64 | 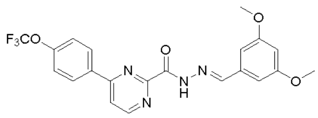 (*E*) -*N*'-(3,5-dimethoxybenzylidene)-4-(4-(trifluoromethoxy)phenyl)pyrimidine-2-carbohydrazide | 400 MHz, DMSO-*d6* δ 11,94 (s, 1H), 9,07 (br d, J = 4,8 Hz, 1H), 8,60 (br s, 1H), 8,49 (br d, J = 7,1 Hz, 2H), 8,26 (br d, J = 3,8 Hz, 1H), 7,57 (br d, J = 8,2 Hz, 2H), 6,93 (br s, 2H), 6,61 (br s, 1H), 3,83 (br s, 6H). [M+H]+: 447,1. |
| 65 | 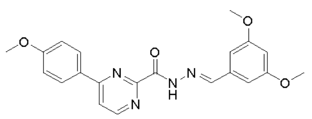 (*E*) -*N*'-(3,5-dimethoxybenzylidene)-4-(4-methoxyphenyl)pyrimidine-2-carbohydrazide | 400 MHz, DMSO-*d6* δ 11,86 (br s, 1H), 8,92 (br d, *J* = 4,6 Hz, 1H), 8,57 (br s, 1H), 8,31 (br d, *J* = 7,1 Hz, 2H), 8,11 (br d, *J* = 4,4 Hz, 1H), 7,12 (br d, *J* = 8,4 Hz, 2H), 6,90 (br s, 2H), 6,58 (br s, 1H), 3,87 (s, 3H), 3,81 (brs, 6H). [M+H]+: 393,1. |

(continued)

| Compound No. | Structure | 1H-NMRI MS m/z [M+H] + |
|---|---|---|
| 66 | 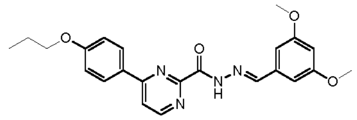<br>(*E*) -*N'*-(3,5-dimethoxybenzylidene)-4-(4-propoxyphenyl)pyrimidine-2-carbohydrazide | 400 MHz, DMSO-*d6* $\delta$ 11,87 (s, 1H), 8,94 (br d, J = 5,1 Hz, 1H), 8,60 (br s, 1H), 8,32 (br d, J = 7,8 Hz, 2H), 8,12 (br d, J = 4,5 Hz, 1H), 7,13 (br d, J = 8,7 Hz, 2H), 6,93 (br s, 2H), 6,61 (br s, 1H), 4,08 (t, J = 6,5 Hz, 2H), 3,83 (br s, 6H), 1,80 (sxt, J = 7,1 Hz, 2H), 1,03 (t, J = 7,4 Hz, 3H). [M+H]+: 421,1. |
| 67 | 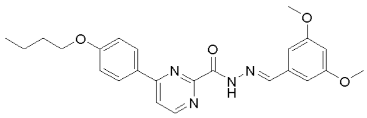<br>(*E*)-4-(4-butoxyphenyl) -*N'*-(3,5-dimethoxybenzylidene)pyrimidine-2-carbohydrazide | 400 MHz, DMSO-*d6* $\delta$ 11,85 (s, 1H), 8,91 (br d, J = 4,9 Hz, 1H), 8,57 (brs, 1H), 8,29 (br d, J = 7,5 Hz, 2H), 8,09 (br d, J = 4,0 Hz, 1H), 7,10 (br d, J = 8,6 Hz, 2H), 6,90 (br s, 2H), 6,58 (br s, 1H), 4,10 (t, J = 6,4 Hz, 2H), 3,81 (br s, 6H), 1,80 - 1,69 (m, 2H), 1,47 (sxt, J = 7,3 Hz, 2H), 0,95 (t, J = 7,4 Hz, 3H). [M+H] +: 435,2. |
| 68 | 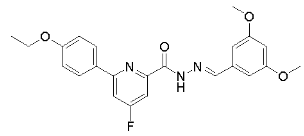<br>(*E*)-*N'*-(3,5-dimethoxybenzylidene)-6-(4-ethoxyphenyl)-4-fluoropicolinohydrazide | 400 MHz, DMSO-*d6* $\delta$ 11.96 (s, 1H), 8.67 (s, 1H), 8.37 (d, *J* = 8.9 Hz, 2H), 8.13 (dd, *J* = 2.1, 10.6 Hz, 1H), 7.78 (dd, *J* = 2.1, 8.8 Hz, 1H), 7.08 (d, J= 8.8 Hz, 2H), 6.93 (d, *J* = 2.1 Hz, 2H), 6.62 (t, *J* = 2.2 Hz, 1H), 4.14 (q, *J* = 7.0 Hz, 2H), 3.82 (s, 6H), 1.38 (t, *J* = 6.9 Hz, 3H). [M+H]+: 424,0. |
| 69 | 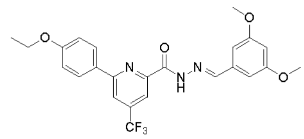<br>(*E*) -*N'*-(3,5-dimethoxybenzylidene)-6-(4-ethoxyphenyl)picolinohydrazide | 400 MHz, DMSO-*d6* $\delta$ 12.05 (s, 1H), 8.66 (s, 1H), 8.51 - 8.40 (m, 3H), 8.13 (s, 1H), 7.08 (d, J=9.0 Hz, 2H), 6.91 (d, J=2.2 Hz, 2H), 6.59 (t, J=2.2 Hz, 1H), 4.13 (q, J=7.0 Hz, 2H), 3.79 (s, 6H), 1.36 (t, J=7.0 Hz, 3H). [M+H]+: 474,1. |
| 70 | 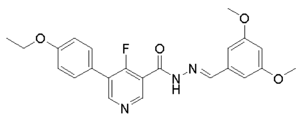<br>(*E*) -*N'*-(3,5-dimethoxybenzylidene)-5-(4-ethoxyphenyl)-4-fluoronicotinohydrazide | |
| 71 | 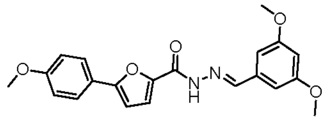<br>(*E*) -*N'*-(3,5-dimethoxybenzylidene)-5-(4-methoxyphenyl)furan-2-carbohydrazide | 400 MHz, DMSO-*d6* $\delta$ 11.77 (s, 1H), 8.44 (br s, 1H), 7.89 (br d, *J* = 6.7 Hz, 2H), 7.37 (br s, 1H), 7.12 - 7.00 (m, 3H), 6.89 (d, *J* = 1.8 Hz, 2H), 6.59 (t, *J* = 1.9 Hz, 1H), 3.82 - 3.80 (m, 1H), 3.91 - 3.74 (m, 8H). [M+H]+: 381,1. |
| 72 | 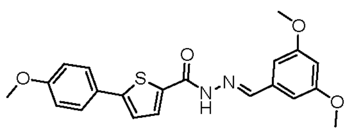<br>(*E*) -*N'*-(3,5-dimethoxybenzylidene)-5-(4-methoxyphenyl)thiophene-2-carbohydrazide | 400 MHz, DMSO-*d6* $\delta$ 11.90 (br s, 1H), 8.50 - 7.82 (m, 2H), 7.68 (br d, *J* = 8.6 Hz, 2H), 7.49 (br s, 1H), 7.04 (br d, *J* = 8.6 Hz, 3H), 6.89 (br s, 1H), 6.58 (t, *J* = 2.0 Hz, 1H), 3.82 (s, 9H). [M+H]+: 397,1. |

(continued)

| Compound No. | Structure | 1H-NMRI MS m/z [M+H] + |
|---|---|---|
| 73 | 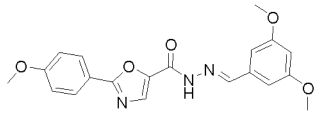 (*E*) -*N*'-(3,5-dimethoxybenzylidene)-2-(4-methoxyphenyl)oxazole-5-carbohydrazide | 400 MHz, DMSO-*d6* δ 12.10 - 11.88 (m, 1H), 8.45 - 7.99 (m, 4H), 7.16 (d, J = 8.7 Hz, 2H), 6.89 (brs, 2H), 6.61 (t, J = 2.1 Hz, 1H), 3.87 (s, 3H), 3.81 (s, 6H). [M+H]+: 382,0. |
| 74 | 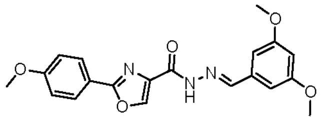 (*E*) -*N'*-(3,5-dimethoxybenzylidene)-2-(4-methoxyphenol)oxazole-4-carbohydrazide | 400 MHz, DMSO-*d6* δ 9.93 (s, 1H), 8.28 (s, 1H), 8.16 (s, 1H), 7.94 (d, *J* = 8.4 Hz, 2H), 6.94 (br d, *J* = 8.4 Hz, 2H), 6.89 (d, *J* = 2.0 Hz, 2H), 6.46 (s, 1H), 3.83 - 3.81 (m, 3H), 3.78 (s, 6H). [M+H]+: 382,1. |
| 75 | 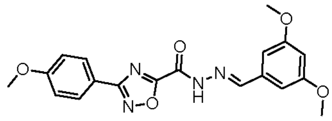 (*E*) -*N*'-(3,5-dimethoxybenzylidene)-3-(4-methoxyphenol)-1,2,4-oxadiazole-5-carbohydrazide | 400 MHz, DMSO-*d6* δ 10.13 (s, 1H), 8.35 (s, 1H), 8.06 (d, *J* = 8.8 Hz, 2H), 7.03 (d, *J* = 8.8 Hz, 2H), 6.96 (d, *J* = 2.2 Hz, 2H), 6.58 (t, *J* = 2.1 Hz, 1H), 3.90 (s, 3H), 3.86 (s, 6H). [M+H]+: 383,1. |
| 76 | 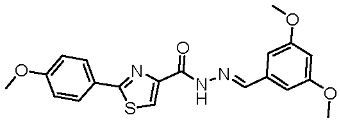 (*E*) -*N*'-(3,5-dimethoxybenzylidene)-2-(4-methoxyphenyl)thiazole-4-carbohydrazide | 400 MHz, DMSO-*d6* δ 10.45 (s, 1H), 8.30 (s, 1H), 8.21 (s, 1H), 7.93 (d, *J* = 8.7 Hz, 2H), 7.01 (d, *J* = 8.8 Hz, 2H), 6.98 (d, *J* = 1.7 Hz, 2H), 6.54 (s, 1H), 3.90 (s, 3H), 3.86 (s, 6H). [M+H]+: 398,0. |
| 77 | 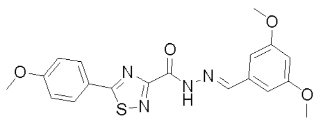 (*E*) -*N*'-(3,5-dimethoxybenzylidene)-5-(4-methoxyphenyl)-1,2,4-th iad iazole-3-carbohydrazide | |

**Example 2. Obtaining Compounds of Formula Ib**

*Compounds 78 -79*

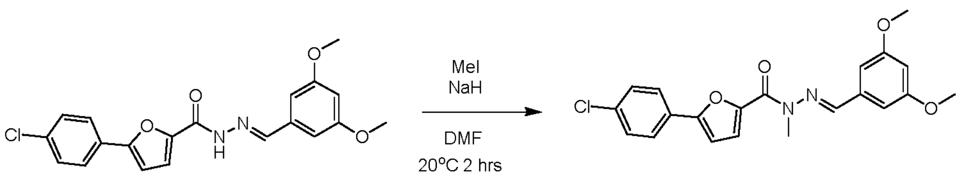


In a flask containing 3.57 mmol of the (*E*)-*N*'-(3,5-dimethoxybenzylidene)-6-(4-methoxyphenyl)pyrazine-2-carbohydrazide, dissolved in 50 mL of DMF, 4.28 mmol of NaH (60% purity) was added at 0 °C. The mixture was heated at 20 °C for 2 hours. Subsequently, 9.86 mmol of methane iodide was added. Subsequently, the reaction mixture was allowed to stir at 20 °C for 2 hours. After total consumption of the starting reagent (monitored by CCF), the reaction mixture was diluted with a solution ofNH4Cl. The product was filtered and used without subsequent purification. 232 mg (90% yield) of the compound of interest were obtained (Table 7).

Table 7. Compounds obtained, from the corresponding reagents, following described method.

| Compound No. | Structure | 1H-NMRI MS m/z [M+H] + |
|---|---|---|
| 78 | 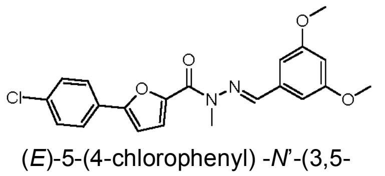 (*E*)-5-(4-chlorophenyl) -*N*'-(3,5-dimethoxybenzylidene)-N-methylfuran-2-carbohydrazide | 400 MHz, DMSO-*d6* $\delta$ 8,06 (1H, s), 7,82 (2H, d), 7,54 (2H, d), 7,49 (1H, d), 7,24 (1H, d), 6,90 (2H, s), 6,56 (1H, s), 3,76 (6H, s), 3,47 (3H, s). |
| 79 | 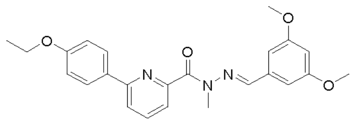 (*E*) -*N*'-(3,5-dimethoxybenzylidene)-6-(4-ethoxyphenyl)-N-methylpicolinohydrazide) | 400 MHz, DMSO-*d6* $\delta$ 8,01 (d, J = 8,8 Hz, 2H), 7,95 - 7,90 (m, 3H), 7,37 (dd, J = 5,5, 3,0 Hz, 1H), 6,99 (d, J = 8,9 Hz, 2H), 6,54 - 6,50 (m, sinais solapados, 2H), 6,40 (t, J = 2,2 Hz, 1H), 4,05 (q, J = 7,0 Hz, 2H), 3,52 (s, 6H), 3,50 (s, 3H), 1,33 (t, J = 7,0 Hz, 3H). [M+H]+: 420,4. |

Example 3. In ***vitro* tests**

Biological assays were performed on Chinese hamster ovary (CHO) cells expressing the human sodium channels Nav 1.8 and Nav 1.7 stably.

The experimental *voltage-clamp* protocol using the whole cell configuration was established on the automated ScreenPatch® 384P platform (SP384PE, Nanion Technologies, Livingston, NJ) and recording of the results was performed with a Nanion 384-well Patch Clamp chip (NPC) (Nanion Technologies, Livingston, NJ).

Compounds of Formula (I), were diluted in eight concentrations in the extracellular solution composed of physiological saline, buffered with HEPES (mM): NaCl, 137; KCl, 4;CaCl2, 3.8; MgCl2, 1; HEPES, 10; Glucose, 10; pH 7.4. The extracellular solution is composed of (mM) CsCl, 50; CsF, 90;MgCl2, 5; EGTA, 5; HEPES, 10; pH 7.2. The duration of exposure of each compound with the cells expressing Nav 1.7 or Nav 1.8 was at least five minutes and the assays were performed at room temperature.

The measurements of the sodium currents of Nav 1.8 and Nav 1.7 were obtained using the voltage protocols described below:

**Protocol 1:** A holding voltage of -90 mV was established followed by a voltage phase of 200 ms to -120 mV, followed by a voltage step of 10 mV for 1 second for Nav 1.8 or 0 mV for Nav 1.7, followed by a step of -100 mV for 20 ms, followed by a step of 20 ms for 10 mV for Nav 1.8 or 0 mV for Nav 1.7 (TP1A) before returning to the holding voltage of -90 mV.

**Protocol 2:** A holding voltage of -100 mV was established followed by an inactivation voltage step at -40 mV for 8 seconds, followed by a -100 mV step for 20 ms, followed by a 20 ms step for 10 mV for Nav 1.8 or 0 mV for Nav 1.7 (TP1A) before returning to the holding voltage of -100 mV.

Protocols were repeated at a frequency of 0.1 Hz (Protocol 1) or 0.05 Hz (Protocol 2) and current amplitude was quantified over the TP1A phase log. The variation in the peak amplitude of the current was evaluated according to the Formula described below, after exposure of the cells expressing the channels, at each concentration of the different compounds:

$$\% \text{ Block} = (1 - (I_{TP1A,molecule} / I_{TP1A,basal}) \times 100\%,$$

where $I_{TP1A,basal}$, and $I_{TP1A,molecule}$ represent the entry peaks of sodium currents into TP1A prior to exposure to the compound and in the presence of the compound, respectively.

The decrease in peak current amplitude, after exposure of the cells to the compounds, was used to calculate the percent relative blocking of the channels relative to the positive control according to the Formula below:

$$\% Block' = 100\% - ((\% Block - \% CP) * (100\% / (\%V - \% CP)),$$

where %V and % CP represent the averages of the values of current inhibition with a vehicle (DMSO) and positive controls, respectively. The sodium currents of the positive control were considered as 100%.

$$\% Block' = (\% 100 / [1 + ([Test] /IC_{50})N],$$

where [Test] represents the concentration of the evaluated molecule, $IC_{50}$ is the concentration of the compound that generates half of the maximum inhibition, N is the Hill's coefficient and % Block' is the percentage of the sodium channel current (Nav 1.8 and Nav 1.7) inhibited at each concentration of the evaluated molecule. Data were obtained by *non-linear least squares* regression with XLfit for Excel (Microsoft, Redmond, WA).

Compounds were tested in at least one assay to obtain the $IC_{50}$ value. For compounds that were tested in two or more assays, the results are described as the average of the $IC_{50}$ values.

Table 8. $IC_{50}$ values for compounds selected from 1-79 in Nav 1.8 and Nav 1.7 channels, Protocol 1.

| Compound No. | Nav1.8 IC50 (nM) | Nav1.7 IC50 (nM) |
|---|---|---|
| 1 | >30000 | >30000 |
| 2 | >30000 | >30000 |
| 3 | >30000 | >30000 |
| 4 | ND | ND |
| 5 | ND | ND |
| 6 | ND | ND |
| 7 | > 6272 | > 16667 |
| 8 | ND | ND |
| 9 | ND | ND |
| 10 | 1370 | 362 |
| 11 | 8580 | 11960 |
| 12 | 3210 | 1891 |
| 13 | ND | ND |
| 16 | 4245 | 4123 |
| 17 | 6825 | 7090 |
| 18 | 6825 | 7090 |
| 19 | 5899 | 5723 |
| 20 | ND | ND |
| 21 | ND | ND |
| 22 | >10000 | >10000 |
| 23 | >10000 | >10000 |
| 24 | 5899 | 5723 |
| 25 | >10000 | >10000 |
| 26 | >10000 | 3502 |
| 27 | >10000 | >10000 |
| 28 | >10000 | >10000 |
| 29 | >10000 | >10000 |

(continued)

| Compound No. | Nav1.8 IC50 (nM) | Nav1.7 IC50 (nM) |
|---|---|---|
| 30 | >10000 | >10000 |
| 32 | 1361 | 1045 |
| 33 | >10000 | >10000 |
| 34 | >10000 | >10000 |
| 35 | >10000 | >10000 |
| 36 | 8425 | 7059 |
| 37 | >10000 | 8759 |
| 42 | >10000 | >10000 |
| 43 | >10000 | >10000 |
| 44 | >10000 | >10000 |
| 45 | >10000 | >10000 |
| 46 | >10000 | >10000 |
| 47 | >10000 | >10000 |
| 48 | >10000 | >10000 |
| 49 | >10000 | >10000 |
| 50 | >10000 | >10000 |
| 51 | >10000 | >10000 |
| 52 | >10000 | >10000 |
| 53 | >10000 | >10000 |
| 55 | >10000 | >10000 |
| 56 | >10000 | >10000 |
| 57 | >10000 | >10000 |
| 58 | >10000 | >10000 |
| 59 | >10000 | >10000 |
| 67 | >10000 | >10000 |
| 78 | >30000 | >30000 |
| 79 | >10000 | >10000 |
| ND = $IC_{50}$ value not available. Percent of inhibition below 10% at a concentration of 10 μM. | | |

Table 9. $IC_{50}$ values for selected compounds between 14-77 in Nav 1.8 and Nav 1.7 channels, Protocol 2

| Compound No. | Nav1.8 IC50 [nM] | Nav1.7 IC50 [nM] |
|---|---|---|
| 14 | 753,6 | 1317 |
| 15 | 297.1 | 272 |
| 31 | 224,4 | 443,5 |
| 38 | >1000 | >10000 |
| 39 | >1000 | >10000 |
| 41 | >1000 | 1200 |

(continued)

| Compound No. | Nav1.8 IC50 [nM] | Nav1.7 IC50 [nM] |
|---|---|---|
| 40 | 140 | 123 |
| 54 | 312,6 | 137,2 |
| 60 | 5044,1 | 5150 |
| 61 | 183,1 | 650,9 |
| 62 | 461, 9 | 301,5 |
| 63 | >1000 | 305,9 |
| 64 | 383,7 | 173,1 |
| 65 | >1000 | 6441,3 |
| 66 | >1000 | 569,2 |
| 68 | 636 | >10000 |
| 69 | >1000 | >10000 |
| 71 | >1000 | >10000 |
| 72 | >1000 | >10000 |
| 73 | >1000 | >10000 |
| 74 | >1000 | >10000 |
| 75 | >1000 | >10000 |
| 76 | >1000 | >10000 |

From these results, the inhibitory activity of Nav 1.7 and/or 1.8 is proven, the application of which is readily carried out by persons skilled in the art in pharmaceutical compositions, which may comprise one or more of said compounds of Formula I, kits, in addition to uses in the treatment of pain-related pathologies.

In particular, such results indicate the possibility of using the compounds of Formula (I) in the preparation of medicaments for treating conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that various modifications may be made by a person skilled in the art thereto without departing from the scope of the present invention. Accordingly, the present invention should not be considered limited to the exemplary embodiments described in the present application.

## Claims

**1.** A COMPOUND **characterized in that** it comprises Formula (I)

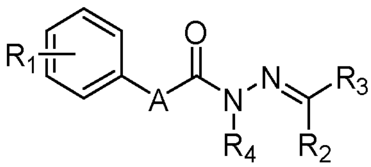

Formula (I)

or a pharmaceutically acceptable salt, hydrate, solvate, and isomer thereof, wherein:
A is selected from the group consisting of

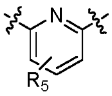 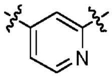 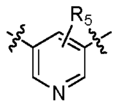 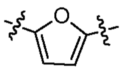 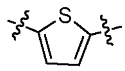 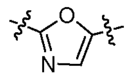 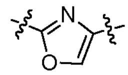

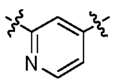 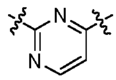 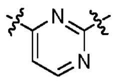 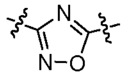 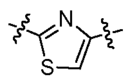 ou 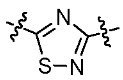

- R1 is selected from the group consisting of hydrogen, halogen, linear or branched C1-6 alkoxy, 2-morpholinoethoxy, C3-6 cycloalkyloxy, or C1-5 haloalkyloxy;
- R2 and R4 are independently selected from the group consisting of hydrogen or branched or linear C1-6alkyl;
- R3 is selected from the group consisting of hydrogen, heterocycle or substituted heterocycle, or R6;
- R5 is selected from the group consisting of hydrogen, halogen, trifluoromethyl.
- R6 is

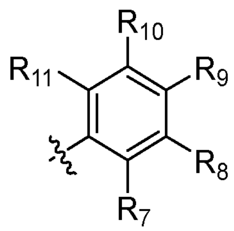

- R7, R8, R9, R10 and R11 are independently selected from the group consisting of hydrogen, halogen, linear or branchedC1-6 alkoxy.

**2.** The COMPOUND according to claim 1, **characterized in that**

- R1 is selected from the group consisting of hydrogen, chloro, bromo, fluoro, methoxy, ethoxy, isopropoxy, propoxy, butoxy, 2-morpholinoethoxy, cyclopropoxy; cyclopentyloxy, cyclohexyloxy, cyclobutyloxy or trifluoromethoxy;
- R2 and R4 are independently selected from the group consisting of hydrogen, methyl, ethyl, propyl or isopropyl;
- R3 is selected from the group consisting of hydrogen, thiophene, furan, 1H-pyrrol-2-yl, 1-methyl-1H-pyrrol-2-yl, 1-ethyl-1H-pyrrol-2-yl, 1H-imidazol-5-yl, 1H-pyrazol-5-yl, 2-oxazole,2-thiazole, 5-oxazole, 5-thiazole, 1,3,4-oxadiazole, 1,3,4-thiadiazole, 3-isoxazole, 5-isoxazole, 3-isothiazole, 5-isothiazole, isoxazolpyridin-3-yl; pyridin-4-yl, pyridin-2-yl; 2-morpholinopyridin-3-yl, 4-((dimethylamino)methyl)thiophen-2-yl orR6;
- R5 is selected from the group consisting of hydrogen, chloro, bromo, fluoro or trifluoromethyl.
- R7, R8, R9, R10 and R11 are independently selected from the group consisting of hydrogen, chlorine, fluorine, bromine, methoxy, ethoxy, propoxy or isoproxy.

**3.** The compound of claim 1 or 2, **characterized in that:**

- R1 is selected from the group consisting of hydrogen, chloro, bromo, fluoro, methoxy, ethoxy, isopropoxy, propoxy, butoxy, 2-morpholinoethoxy, cyclopropoxy; cyclopentyloxy, cyclohexyloxy, cyclobutyloxy or trifluoromethoxy;
- R2 and R4 are independently selected from hydrogen or methyl;
- R3 is selected from the group consisting of hydrogen, thiophene, pyridin-3-yl; pyridin-4-yl, pyridin-2-yl; 2-morpholinopyridin-3-yl, 4-((dimethylamino)methyl)thiophen-2-yl, or R6;
- R5 is selected from the group consisting of hydrogen, chloro, bromo, fluoro or trifluoromethyl.
- R7 is selected from hydrogen, chlorine, fluorine or methoxy;
- R8 and R10 are independently selected from hydrogen or methoxy;
- R9 is hydrogen;
- R11 is selected from the group consisting of hydrogen, chlorine or fluorine.

**4.** The COMPOUND according to any one of claims 1 to 3, **characterized in that** it is selected from the group consisting of:

(*E*)-5-(4-chlorophenyl)-*N*'-(thiophen-2-ylmethylene)furan-2-carbohydrazide (Compound 1);
(E)-5-(4-chlorophenyl)-N'-(3,5-dimethoxybenzylidene)furan-2-carbohydrazide (Compound 2);
(*E*)-5-(4-ethoxyphenyl)-*N'*-(thiophen-2-ylmethylene)furan-2-carbohydrazide (Compound 3);
(*E*)-5-(4-ethoxyphenyl)-*N'*-((2-morpholinopyridin-3-yl)methylene)furan-2-carbohydrazide (Compound 4);
(*E*)-*N'*-((4-((dimethylamino)methyl)thiophen-2-yl)methylene)-5-(4-ethoxyphenyl)furan-2-carbohydrazide (Compound 5);
(*E*)-5-(4-ethoxyphenyl)-*N*'-(pyridin-3-ylmethylene)furan-2-carbohydrazide (Compound 6);
(*E*)-5-(4-isopropoxyphenyl)-*N*'-(thiophen-2-ylmethylene)furan-2-carbohydrazide (Compound 7);
(*E*)-*N'*-((4-((dimethylamino)methyl)thiophen-2-yl)methylene)-5-(4-isopropoxyphenyl)furan-2-carbohydrazide (Compound 8);
(*E*)-5-(4-(2-morpholinoethoxy)phenyl)-*N*'-(thiophen-2-ylmethylene)furan-2-carbohydrazide (Compound 9);
(*E*)-6-(4-chlorophenyl)-*N*'-(3,5-dimethoxybenzylidene)picolinohydrazide (Compound 10);
(*E*)-6-(4-chlorophenyl)-*N'*-(thiophen-2-ylmethylene)picolinohydrazide (Compound 11);
(*E*)-6-(4-ethoxyphenyl)-*N*'-(thiophen-2-ylmethylene)picolinohydrazide (Compound 12);
(*E*)-6-(4-isopropoxyphenyl)-*N*'-(thiophen-2-ylmethylene)picolinohydrazide (Compound 13);
(*E*) -*N'*-(3,5-dimethoxybenzylidene)-6-(4-methoxyphenyl)picolinohydrazide (Compound 14);
(*E*)-*N'*-(3,5-dimethoxybenzylidene)-6-(4-propoxyphenyl)picolinohydrazide (Compound 15);
(*E*)-5-(4-chlorophenyl)-*N'*-(3,5-dimethoxybenzylidene)nicotinohydrazide (Compound 16);
(*E*)-*N'*-(3,5-dimethoxybenzylidene)-5-(4-(trifluoromethoxy)phenyl)nicotinohydrazide (Compound 17);
(*E*)-*N'*-(3,5-dimethoxybenzylidene)-5-(4-ethoxyphenyl)nicotinohydrazide (Compound 18);
(*E*)-*N'*-(3,5-dimethoxybenzylidene)-5-(4-isopropoxyphenyl)nicotinohydrazide (Compound 19);
(*E*)-2-(4-chlorophenyl)-*N*'-(3,5-dimethoxybenzylidene)isonicotinohydrazide (Compound 20);
(*E*)-*N*'-(3,5-dimethoxybenzylidene)-2-(4-(trifluoromethoxy)phenyl)isonicotinohydrazide (Compound 21);
(*E*)-*N*'-(3,5-dimethoxybenzylidene)-2-(4-ethoxyphenyl)isonicotinohydrazide (Compound 22);
(*E*)-*N*'-(3,5-dimethoxybenzylidene)-2-(4-isopropoxyphenyl)isonicotinohydrazide (Compound 23);
(*E*)-*N*'-(3,5-dimethoxybenzylidene)-6-(4-isopropoxyphenyl)picolinohydrazide (Compound 24);
(*E*)-*N*'-(3,5-dimethoxybenzylidene)-5-(4-methoxyphenyl)nicotinohydrazide (Compound 25);
(*E*)-*N'*-(3,5-dimethoxybenzylidene)-5-(4-propoxyphenyl)nicotinohydrazide (Compound 26);
(*E*)-5-(4-butoxyphenyl)-*N*'-(3,5-dimethoxybenzylidene)nicotinohydrazide (Compound 27);
(*E*)-5-(4-(cyclopentyloxy)phenyl)-*N*'-(3,5-dimethoxybenzylidene)nicotinohydrazide (Compound 28);
(*E*)-*N*'-(3,5-dimethoxybenzylidene)-2-(4-methoxyphenyl)isonicotinohydrazide (Compound 29);
(*E*)-*N*'-(3,5-dimethoxybenzylidene)-2-(4-propoxyphenyl)isonicotinohydrazide (Compound 30);
(*E*)-*N*'-(3,5-dimethoxybenzylidene)-6-(4-ethoxyphenyl)picolinohydrazide (Compound 31);
(*E*)-6-(4-ethoxyphenyl)-*N*'-(2-fluorobenzylidene)picolinohydrazide (Compound 32);
(*E*)-*N*'-(2-chlorobenzylidene)-6-(4-ethoxyphenyl)picolinohydrazide (Compound 33);
(*E*)-*N'*-(2,6-dichlorobenzylidene)-6-(4-ethoxyphenyl)picolinohydrazide (Compound 34);
(*E*)-*N'*-(2-chloro-6-fluorobenzylidene)-6-(4-ethoxyphenyl)picolinohydrazide (Compound 35);
(*E*)-6-(4-ethoxyphenyl)-*N'*-(2-methoxybenzylidene)picolinohydrazide (Compound 36);
(E)-6-(4-ethoxyphenyl)-*N'*-((2-morpholinopyridin-3-yl)methylene)picolinohydrazide (Compound 37);
(*E*)-2-(4-butoxyphenyl)-*N'*-(3,5-dimethoxybenzylidene)isonicotinohydrazide (Compound 38);
(*E*)-2-(4-(cyclopentyloxy)phenyl)-*N'*-(3,5-dimethoxybenzylidene)isonicotinohydrazide (Compound 39);
(*E*)-*N'*-(3,5-dimethoxybenzylidene)-6-(4-(trifluoromethoxy)phenyl)picolinohydrazide (Compound 40);
(*E*)-6-(4-butoxyphenyl) -*N'*-(3,5-dimethoxybenzylidene)picolinohydrazide (Compound 41);
(*E*)-4-(4-chlorophenyl)-*N*'-(3,5-dimethoxybenzylidene)picolinohydrazide (Compound 42);
(*E*)-4-(4-chlorophenyl)-*N*'-(thiophen-2-ylmethylene)picolinohydrazide (Compound 43);
(*E*)-*N*'-(3,5-dimethoxybenzylidene)-4-(4-ethoxyphenyl)picolinohydrazide (Compound 44);
(*E*)-4-(4-ethoxyphenyl)-*N*'-(thiophen-2-ylmethylene)picolinohydrazide (Compound 45);
(*E*)-*N*'-(3,5-dimethoxybenzylidene)-4-(4-isopropoxyphenyl)picolinohydrazide (Compound 46);
(*E*)-4-(4-isopropoxyphenyl)-*N*'-(thiophen-2-ylmethylene)picolinohydrazide (Compound 47);
(*E*)-4-(4-(cyclopentyloxy)phenyl)-*N*'-(3,5-dimethoxybenzylidene)picolinohydrazide (Compound 48);
(*E*)-*N*'-(3,5-dimethoxybenzylidene)-4-(4-(trifluoromethoxy)phenyl)picolinohydrazide (Compound 49);
(*E*)-4-(4-(cyclopentyloxy)phenyl) -*N* '-(thiophen-2-ylmethylene)picolinohydrazide (Compound 50);
(*E*)-*N*'-(thiophen-2-ylmethylene)-4-(4-(trifluoromethoxy)phenyl)picolinohydrazide (Compound 51);
(*E*)-2-(4-chlorophenyl)-*N*'-(3,5-dimethoxybenzylidene)pyrimidine-4-carbohydrazide (Compound 52);
(*E*)-*N*'-(3,5-dimethoxybenzylidene)-2-(4-ethoxyphenyl)pyrimidine-4-carbohydrazide (Compound 53);
(*E*)-*N*'-(3,5-dimethoxybenzylidene)-2-(4-isopropoxyphenyl)pyrimidine-4-carbohydrazide (Compound 54);
(*E*)-2-(4-(cyclopentyloxy)phenyl)-*N*'-(3,5-dimethoxybenzylidene)pyrimidine-4-carbohydrazide (Compound 55 ;
(*E*)-*N*'-(3,5-dimethoxybenzylidene)-2-(4-(trifluoromethoxy)phenyl)pyrimidine-4-carbohydrazide (Compound

56);
(*E*)-*N*'-(3,5-dimethoxybenzylidene)-2-(4-methoxyphenyl)pyrimidine-4-carbohydrazide (Compound 57);
(*E*)-*N'*-(3,5-dimethoxybenzylidene)-2-(4-propoxyphenyl)pyrimidine-4-carbohydrazide (Compound 58);
(*E*)-2-(4-butoxyphenyl)-*N*'-(3,5-dimethoxybenzylidene)pyrimidine-4-carbohydrazide (Compound 59);
(*E*)-4-(4-chlorophenyl)-*N*'-(3,5-dimethoxybenzylidene)pyrimidine-2-carbohydrazide (Compound 60);
(*E*)-*N*'-(3,5-dimethoxybenzylidene)-4-(4-ethoxyphenyl)pyrimidine-2-carbohydrazide (Compound 61);
(*E*)-*N*'-(3,5-dimethoxybenzylidene)-4-(4-isopropoxyphenyl)pyrimidine-2-carbohydrazide (Compound 62);
(*E*)-4-4-(cyclopentyloxy)phenyl)-*N*'-(3,5-dimethoxybenzylidene)pyrimidine-2-carbohydrazide (Compound 63);
*(E)*-*N*'-(3,5-dimethoxybenzylidene)-4-(4-(trifluoromethoxy)phenyl)pyrimidine-2-carbohydrazide (Compound 64);
(*E*)-*N*'-(3,5-dimethoxybenzylidene)-4-(4-methoxyphenyl)pyrimidine-2-carbohydrazide(Compound 65);
*(E)*-*N*'-(3,5-dimethoxybenzylidene)-4-(4-propoxyphenyl)pyrimidine-2-carbohydrazide (Compound 66);
(*E*)-4-(4-butoxyphenyl)-*N*'-(3,5-dimethoxybenzylidene)pyrimidine-2-carbohydrazide (Compound 67);
(*E*)-*N*'-(3,5-dimethoxybenzylidene)-6-(4-ethoxyphenyl)-4-fluoropicolinohydrazide (Compound 68);
(*E*)-*N*'-(3,5-dimethoxybenzylidene)-6-(4-ethoxyphenyl)-4-trifluoromethyl)picolinohydrazide (Compound 69);
(*E*)-*N*'-(3,5-dimethoxybenzylidene)-5-(4-ethoxyphenyl)-4-fluoronicotinohydrazide (Compound 70);
(*E*)-*N*'-(3,5-dimethoxybenzylidene)-5-(4-methoxyphenyl)furan-2-carbohydrazide (Compound 71);
(*E*)-*N*'-(3,5-dimethoxybenzylidene)-5-(4-methoxyphenyl)thiophene-2-carbohydrazide (Compound 72);
(*E*)-*N'*-(3,5-dimethoxybenzylidene)-2-(4-methoxyphenyl)oxazole-5-carbohydrazide (Compound 73);
(*E*)-*N*'-(3,5-dimethoxybenzylidene)-2-(4-methoxyphenol)oxazole-4-carbohydrazide (Compound 74);
(*E*)-*N*'-(3,5-dimethoxybenzylidene)-3-(4-methoxyphenol)-1,2,4-oxadiazole-5-carbohydrazide (Compound 75);
(*E*)-*N*'-(3,5-dimethoxybenzylidene)-2-(4-methoxyphenyl)thiazole-4-carbohydrazide (Compound 76);
(*E*)-*N*'-(3,5-dimethoxybenzylidene)-5-(4-methoxyphenyl)-1,2,4-thiadiazole-3-carbohydrazide (Compound 77);
(*E*)-5-(4-chlorophenyl)-*N*'-(3,5-dimethoxybenzylidene)-N-methylfuran-2-carbohydrazide (Compound 78);
(*E*)-*N'*-(3,5-dimethoxybenzylidene)-6-(4-ethoxyphenyl)-N-methylpicolinohydrazide (Compound 79).

**5.** The COMPOUND according to any one of claims 1 to 4, **characterized in that** it is a selective inhibitor of the voltage-gated sodium channels Nav 1.7 and/or Nav 1.8.

**6.** A PHARMACEUTICAL COMPOSITION **characterized in that** it comprises a therapeutically effective amount of one or more compounds of Formula (I) or a pharmaceutically acceptable salt, hydrate, solvate and isomer thereof, as defined in any one of claims 1 to 5; and one or more pharmaceutically acceptable excipients.

**7.** The PHARMACEUTICAL COMPOSITION according to claim 6, **characterized in that** it is formulated as an oral, sublingual, nasal, parenteral, injectable, submuscular, topical, transdermal, ocular or rectal composition.

**8.** USE OF COMPOUND(S) OF FORMULA (I), as defined in any one of claims 1 to 5, **characterized in that** it is for preparing a medicament for treating pathologies related to neuropathic pain.

**9.** The USE according to claim 8, **characterized in that** said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

**10.** A METHOD OF TREATING, PREVENTING, ALLEVIATING, SUPPRESSING, AND/OR CONTROLLING neuropathic pain-related pathologies **characterized by** administering an effective amount of at least one compound of Formula (I) or a pharmaceutically acceptable salt, hydrate, solvate, and isomer thereof as defined in any one of claims 1 to 5.

**11.** THE METHOD of claim 10 **characterized in that** it is for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**12.** THE METHOD according to claim 10 or 11, **characterised in** administration of the at least one compound of Formula (I) is selected from the group comprising orally, sublingually, nasally, parenterally, injectable, submuscularly, topically, transdermally, ocularly, and rectally.

**13.** A PROCESS FOR OBTAINING A COMPOUND OF FORMULA (I), as defined in any one of claims 1 to 5, **characterized in that** it comprises the steps:

(a) Forming the intermediate of Formula III:

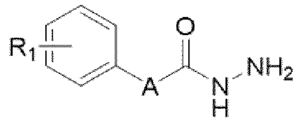

Formula III

from the hydrazinolysis reaction of an intermediate of Formula IV:

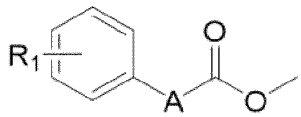

Formula IV

(b) obtaining a Formula I compound wherein R4 is hydrogen;

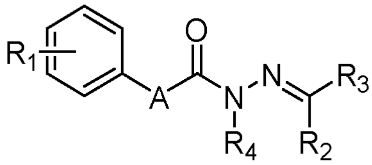

Formula I

from condensation of intermediates of Formula II:

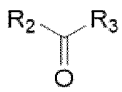

Formula II:

and Formula III, with or without presence of catalyst and a suitable solvent; wherein:
A is selected from the group consisting of

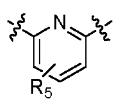 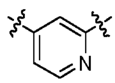 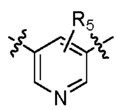 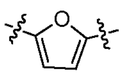 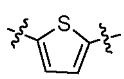 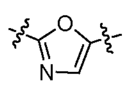 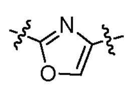

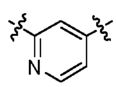 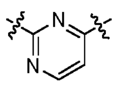 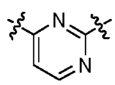 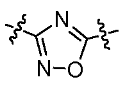 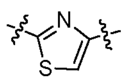 or 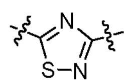

- R1 is selected from the group consisting of hydrogen, halogen, linear or branched C1-6 alkoxy, 2-morpholinoethoxy, C3-6 cycloalkyloxy, or C1-5 haloalkyloxy;
- R2 and R4 are independently selected from the group consisting of hydrogen or branched or linear C1-6 alkyl;
- R3 is selected from the group consisting of hydrogen, heterocycle or substituted heterocycle, or R6;
- R5 is selected from the group consisting of hydrogen, halogen, trifluoromethyl.
- R6 is

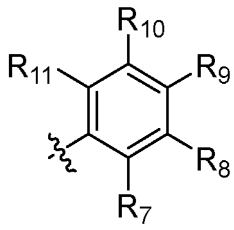

- R7,R8, R9, R10 andR11 are independently selected from the group consisting of hydrogen, halogen, linear or branchedC1-6 alkoxy .

**14.** The PROCESS of claim 13, further **characterised in that** it further comprises a step (c) of forming a compound of Formula (I) wherein R4 is linear or branched C1-5 alkyl from the nucleophilic substitution reaction of a compound obtained in step (b), with linear or branched C1-6 alkyl halides in the presence of inorganic base and polar aprotic solvents.

**15.** The PROCESS, according to claim 13 or 14, **characterized in that** in step (b) said catalyst is selected from concentrated hydrochloric acid, acetic acid, trifluoroacetic acid or formic acid and said solvent is selected from dimethylformamide, dimethylsulfoxide, alcohols, or combinations thereof.

**16.** The PROCESS according to any one of claims 13 to 15, **characterized in that** in step (c) said inorganic base is selected from K2CO3 or NaH.

**17.** A KIT **characterized in that** it comprises a pharmaceutical composition as defined in claim 6; and an application device.

**INTERNATIONAL SEARCH REPORT**

International application No. PCT/BR2022/050304

A. CLASSIFICATION OF SUBJECT MATTER **IPC: A61K 31/175 (2006.01), A61K 31/505 (2006.01), A61K 31/44 (2006.01), A61K 31/34 (2006.01), A61K 31/381 (2006.01), A61K 31/42 (2006.01), A61K 31/4245 (2006.01), A61K 31/425 (2006.01), A61K 31/433 (2006.01), A61P 25/02 (2006.01), A61P 29/00 (2006.01);** See CPC classification in Supplemental Box.

According to International Patent Classification (IPC) or to both national classification and IPC

B. FIELDS SEARCHED

Minimum documentation searched (classification system followed by classification symbols)

**A61K 31, A61P 25, A61P 29**

Documentation searched other than minimum documentation to the extent that such documents are included in the fields searched

**Banco de dados INPI-BR; Periódicos Capes**

Electronic data base consulted during the international search (name of data base and, where practicable, search terms used)

**ESPACENET; CASLINK (REGISTRY, MARPAT, CAPLUS: STN); GOOGLE PATENTS; PUBMED.**

C. DOCUMENTS CONSIDERED TO BE RELEVANT

| Category* | Citation of document, with indication, where appropriate, of the relevant passages | Relevant to claim No. |
|---|---|---|
| X | CN 1752085 A (INST OF TOXIC PHARMACOLOGY ACA [CN]) 29 March 2006 (2006-03-29) See abstract; pages 8-13; claim 1. | 1-8, 13-17 |
| Y | | 9 |
| X | WO 2020209932 A1 (UNIV PUERTO RICO [US]) 15 October 2020 (2020-10-15) See paragraphs [0035]-[0068]; claim 1. | 1-7 |
| Y | | 8, 9, 13-17 |
| X | CN 111925357 A (KUNMING INST BOTANY CAS) 13 November 2020 (2020-11-13) See abstract; paragraphs [0010]-[0086], [0123]-[0794]; claim 1. | 1-8, 13-17 |
| Y | | 9 |

[x] Further documents are listed in the continuation of Box C. [x] See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **23/09/2022** | 28/09/2022 |
| Name and mailing address of the ISA/ BR | Authorized officer |
| Facsimile No. | Telephone No. |

Form PCT/ISA/210 (second sheet) (January 2015)

**INTERNATIONAL SEARCH REPORT**

International application No.
PCT/BR2022/050304

C (Continuation). DOCUMENTS CONSIDERED TO BE RELEVANT

| Category* | Citation of document, with indication, where appropriate, of the relevant passages | Relevant to claim No. |
|---|---|---|
| X | WO 2019227771 A1 (QINGDAO KINGAGROOT CHEMICAL COMPOUND CO LTD [CN]) 05 December 2019 (2019-12-05) | 1-7, 13-17 |
| Y | See abstract; pages 1-26; claim 1. | 8, 9 |
| X | WO 2008121877 A2 ( PEACH JOANNE [GB]) 09 October 2008 (2008-10-09) | 1-8, 13-17 |
| Y | See paragraphs [0011], [0055], [0056], [0086], [0087], [0121], [0138]; claim 1. | 9 |
| X | WO 2004105488 A1 ( MARAVETZ LESTER L [US]) 09 December 2004 (2004-12-09) | 1-7, 13-15, 17 |
| Y | See abstract; pages 1-13; claim 1. | 8, 9, 16 |
| X | US 2010035932 A1 (SCHEPETKIN, I.A. *et al.* [US]) 11 February 2010 (2010-02-11) | 1-8, 17 |
| Y | See paragraphs [0008]-[0012], [0060]-[0081], [0116], [0120], claims 1, 26, 27. | 9, 13-16 |
| X | WO 2020023802 A1 (HOPE CITY [US]) 30 January 2020 (2020-01-30) | 1-8, 17 |
| Y | See abstract; paragraphs [0115], [0116]-[0161]; claim 1. | 9, 13-16 |
| X | WO 2013123071 A1 (CLEAVE BIOSCIENCES INC [US]) 22 August 2013 (2013-08-22) | 1-8, 17 |
| Y | See abstract; page 80; claim 1. | 9, 13-16 |
| Y | Lopes A.B. *et al.* Characterization of amide bond conformers for a novel heterocyclic template of N-acylhydrazone derivatives. Molecules. 2013 Sep 25;18(10):11683-704. doi: 10.3390/molecules181011683. PMID: 24071978; PMCID: PMC6270085. See the whole document. | 1-9, 13-17 |

Form PCT/ISA/210 (continuation of second sheet) (January 2015)

**INTERNATIONAL SEARCH REPORT**

International application No. PCT/BR2022/050304

C (Continuation). DOCUMENTS CONSIDERED TO BE RELEVANT

| Category* | Citation of document, with indication, where appropriate, of the relevant passages | Relevant to claim No. |
|---|---|---|
| Y | Da Silva Y.K. et al. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893.<br><br>See the whole document, especially abstract and pages 5007-5008, 5014. | 1-9, 13-17 |
| Y | US 2008300240 A1 (OMEROS CORP [US])<br>04 December 2008 (2008-12-04)<br><br>See the whole document, especially p. 2-10, Example 7 and claims 1, 2 and 10. | 1-9, 13-17 |
| Y | AR 049472 A1 ( ASTRAZENECA AB [SE])<br>09 August 2006 (2006-08-09)<br><br>See abstract from document and also abstract from MARPAT base (accession number: 154:486357). | 1-9, 13-17 |
| Y | Brokaite, K. *et al.* Synthesis and Investigation of Some 1,4-Disubstituted 2-Pyrrolidinones. Chemistry of Heterocyclic Compounds. 42. 1158-1167, 2006. doi: 10.1007/s10593-006-0220-1.<br><br>See the whole document. | 1-7, 17 |
| A | | 8, 9, 13-16 |
| Y | Intaitė, V. *et al.* Synthesis and structure of new 1,3-disubstituted 5-oxopyrrolidine derivatives. Chemija. 23. 52-60, 2012. http://mokslozurnalai.lmaleidykla.lt/publ/0235-7216/2012/1/52-60.pdf<br><br>See the whole document. | 1-7, 17 |
| A | | 8, 9, 13-16 |
| Y | GAL, M.; TIHANYI, E.; DVORTSAK, P.. Ring-Chain Tautomerism of ortho-Amino-Substituted Aromatic Carboxylic Acid Hydrazones. Part 1. Formation of 1,2,3,4-Tetrahydro-5H-1,3,4-benzotriazepin-5-ones and 5,6,7,8-Tetrahydro-4H-pyrazolo(3,4-e)(1,2,4)triazepin-4-ones. Acta Chimica Hungarica, 123(1-2), 55-61, 1986. Resumo também publicado em: ChemInform 1987, 18, No. 22, Abstract 220.<br><br>See abstracts from CAPLUS base (accession number: 1987:576008) and from ChemInform review. | 1-7, 17 |
| A | | 8, 9, 13-16 |

Form PCT/ISA/210 (continuation of second sheet) (January 2015)

INTERNATIONAL SEARCH REPORT

International application No.
PCT/BR2022/050304

C (Continuation). DOCUMENTS CONSIDERED TO BE RELEVANT

| Category* | Citation of document, with indication, where appropriate, of the relevant passages | Relevant to claim No. |
|---|---|---|
| Y | Milcent, R. *et al*. Synthesis of 5-aryl-3-carbazoyl-1,3,4-oxadiazol-2(3H)-one. Derivatives and their ring transformation into 5-benzamido-1,2,4-triazolidine-3,5-dione derivatives. Journal of Heterocyclic Chemistry (1989), 26(1), 231-6. https://doi.org/10.1002/jhet.5570260141 . Abstract also published on ChemInform 1989, 20, No. 34, Abstract 184. See page 1 and abstracts from CAPLUS base (accession number: 1989:457644) and from ChemInform review. | 1-7, 17 |
| A | | 8, 9, 13-16 |
| Y | JP 2001139566 A (NIHON NOHYAKU CO LTD) 22 May 2001 (2001-05-22) | 1-7, 17 |
| A | | 8, 9, 13-16 |
| Y | WO 2004094370 A2 ( BUTLER MICHELLE M [US]) 04 November 2004 (2004-11-04) See abstract and claim 1. | 1-7, 17 |
| A | | 8, 9, 13-16 |
| Y | WO 2017184999 A1 (DANA FARBER CANCER INST INC [US]) 26 October 2017 (2017-10-26) See abstract and claim 1. | 1-7, 17 |
| A | | 8, 9, 13-16 |

Form PCT/ISA/210 (continuation of second sheet) (January 2015)

**INTERNATIONAL SEARCH REPORT**

International application No.
PCT/BR2022/050304

**Box No. II Observations where certain claims were found unsearchable (Continuation of item 2 of first sheet)**

This international search report has not been established in respect of certain claims under Article 17(2)(a) for the following reasons:

1. [X] Claims Nos.: **10-12**
because they relate to subject matter not required to be searched by this Authority, namely:

Claims 10-12 contain subject matter that falls under the provisions of PCT Rule 39.1(iv), regarding methods for treatment of the human or animal body by surgery or therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (January 2015)

**INTERNATIONAL SEARCH REPORT**
Information on patent family members

International application No.
PCT/BR2022/050304

| | | | |
|---|---|---|---|
| CN 1752085 A | 2006-03-29 | CN 100355748 C | 2007-12-19 |
| | | WO 2006032173 A1 | 2006-03-30 |
| WO 2020209932 A1 | 2020-10-15 | NONE | |
| CN 111925357 A | 2020-11-13 | NONE | |
| WO 2019227771 A1 | 2019-12-05 | CN 108774179 A | 2018-11-09 |
| WO 2008121877 A2 | 2008-10-09 | WO 2008121877 A3 | 2008-12-31 |
| | | BR PI0809498 A2 | 2014-09-23 |
| | | CN 101668732 A | 2010-03-10 |
| | | EP 2142498 A2 | 2010-01-13 |
| | | JP 2010523579 A | 2010-07-15 |
| | | KR 20100016073 A | 2010-02-12 |
| | | TW 200845957 A | 2008-12-01 |
| | | TW I351949 B | 2011-11-11 |
| | | US 2008269206 A1 | 2008-10-30 |
| | | US 8283351 B2 | 2012-10-09 |
| WO 2004105488 A1 | 2004-12-09 | AR 045688 A1 | 2005-11-09 |
| | | AU 2004243492 A1 | 2004-12-09 |
| | | BR PI0410626 A | 2006-06-20 |
| | | CL 2004001296 A1 | 2005-04-08 |
| | | CN 1842272 A | 2006-10-04 |
| | | EP 1631143 A1 | 2006-03-08 |
| | | JP 2006528955 A | 2006-12-28 |
| | | KR 20060019558 A | 2006-03-03 |
| | | MX PA05012226 A | 2006-02-10 |
| | | US 2007066627 A1 | 2007-03-22 |
| | | ZA 200510406 B | 2008-12-31 |
| US 2010035932 A1 | 2010-02-11 | NONE | |
| WO 2020023802 A1 | 2020-01-30 | US 2021355103 A1 | 2021-11-18 |
| WO 2013123071 A1 | 2013-08-22 | NONE | |
| US 2008300240 A1 | 2008-12-04 | US 7786139 B2 | 2010-08-31 |
| | | EP 2083819 A2 | 2009-08-05 |
| | | ES 2624791 T3 | 2017-07-17 |
| | | US 2011021509 A1 | 2011-01-27 |
| | | US 8278327 B2 | 2012-10-02 |
| | | WO 2008064342 A2 | 2008-05-29 |
| AR 049472 A1 | 2006-08-09 | AT 483706 T | 2010-10-15 |
| | | AU 2005270208 A1 | 2006-02-09 |
| | | BR PI0507497 A | 2007-07-10 |
| | | CA 2555566 A1 | 2006-02-09 |
| | | CN 1984907 A | 2007-06-20 |
| | | CN 101096368 A | 2008-01-02 |
| | | CY 1110965 T1 | 2015-06-11 |
| | | DE 602005023963 D1 | 2010-11-18 |

**INTERNATIONAL SEARCH REPORT**
Information on patent family members

International application No.
PCT/BR2022/050304

| | | | |
|---|---|---|---|
| | | DK 1723144 T3 | 2011-01-03 |
| | | EP 2311830 A1 | 2011-04-20 |
| | | ES 2352110 T3 | 2011-02-15 |
| | | HK 1099285 A1 | 2007-08-10 |
| | | HR P20100654 T1 | 2011-01-31 |
| | | IL 177394 D0 | 2006-12-10 |
| | | JP 2007523168 A | 2007-08-16 |
| | | JP 4912157 B2 | 2012-04-11 |
| | | MY 148488 A | 2013-04-30 |
| | | NO 20063599 L | 2006-10-27 |
| | | NZ 548954 A | 2009-07-31 |
| | | PL 1723144 T3 | 2011-02-28 |
| | | RS 51516 B | 2011-06-30 |
| | | RU 2006128446 A | 2008-03-27 |
| | | RU 2370495 C2 | 2009-10-20 |
| | | SG 146657 A1 | 2008-10-30 |
| | | SI 1723144 T1 | 2011-01-31 |
| | | TW 200602041 A | 2006-01-16 |
| | | UA 84318 C2 | 2008-10-10 |
| | | US 2005272779 A1 | 2005-12-08 |
| | | US 7585881 B2 | 2009-09-08 |
| | | US 2007179188 A1 | 2007-08-02 |
| | | US 2007293545 A1 | 2007-12-20 |
| | | US 2008015204 A1 | 2008-01-17 |
| | | US 2008015234 A1 | 2008-01-17 |
| | | US 2008045571 A1 | 2008-02-21 |
| | | UY 28766 A1 | 2005-08-31 |
| | | WO 2006014185 A1 | 2006-02-09 |
| | | ZA 200606551 B | 2007-11-28 |
| JP 2001139566 A | 2001-05-22 | NONE | |
| WO 2004094370 A2 | 2004-11-04 | WO 2004094370 A3 | 2005-03-03 |
| | | AU 2003303953 A1 | 2004-11-19 |
| | | CA 2497410 A1 | 2004-11-04 |
| | | EP 1549143 A2 | 2005-07-06 |
| | | JP 2006508178 A | 2006-03-09 |
| | | US 2006258729 A1 | 2006-11-16 |
| WO 2017184999 A1 | 2017-10-26 | AU 2017252460 A1 | 2018-10-11 |
| | | CA 3018270 A1 | 2017-10-26 |
| | | CN 109069508 A | 2018-12-21 |
| | | EP 3445365 A1 | 2019-02-27 |
| | | JP 2019514884 A | 2019-06-06 |
| | | US 2019135796 A1 | 2019-05-09 |
| | | US 2020039968 A9 | 2020-02-06 |
| | | US 10633371 B2 | 2020-04-28 |

Form PCT/ISA/210 (patent family annex) (January 2015)

INTERNATIONAL SEARCH REPORT

International application No. PCT/BR2022/050304

In case the space in preceding Box is not sufficient:

Continuation of: CPC Classification

**CPC**: A61K 31/175, A61K 31/505, A61K 31/44, A61K 31/34, A61K 31/381, A61K 31/42, A61K 31/4245, A61K 31/425, A61K 31/433, A61P 25/02, A61P 29/00.

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B


### Non-patent literature cited in the description


- **SCHAIBLE.** *Langenbecks Arch. Surg.,* 2004, vol. 389, 237
- **SMITH.** *Pain,* 2020, vol. 161 (1), S127
- **CAVALLI.** *Int. J. Immunopathol. Pharmacol.,* 2019, vol. 33
- **BOUHASSIRA.** *Rev Neurol (Paris),* 2019, vol. 175 (1-2), 16
- **SCHOLZ.** *Nature Neurosci.,* 2002, vol. 5, 1062
- **COSTIGAN.** *Annu. Rev. Neurosci.,* 2009, vol. 32, 1
- **DWORKIN.** *Clin. J. Pain,* 2002, vol. 18 (6), 343
- **DUCREUX.** *Brain,* 2006, vol. 129, 963
- **PAK.** *Curr. Pain Headache Rep,* 2018, vol. 22 (2), 9
- **KUSHNAREV.** *Expert Opin. Investig. Drugs,* 2020, vol. 29 (3), 259
- **EMERY.** *Expert Opin. Ther. Targets,* 2016, vol. 20 (8), 975
- **CATTERALL.** *Nat. Chem. Biol.,* 2020, vol. 16, 1314
- **WISEDCHAISRI.** *Cell,* 2019, vol. 178 (4), 993
- **CLAIRFEUILLE.** *Science,* 2019, vol. 363, 1302
- **LERA-RUIZ.** *J. Med. Chem.,* 2015, vol. 58 (18), 7093
- **BAGAL.** *J. Med. Chem.,* 2013, vol. 56 (3), 593
- **BAGAL.** *Channels,* 2015, vol. 9 (6), 360
- **LAW.** *Drug Discovery Today,* 2019, vol. 24 (7), 1389
- **BAGAL.** *Channels (Austin),* 2015, vol. 9 (6), 360
- **VETTER.** *Pharmacology & Therapeutics,* 2017, vol. 172, 73
- **AHUJA.** *Science,* 2015, vol. 350 (6267), 1491
- **KINGWELL.** *Nat. Rev. Drug Discov.,* 2019, vol. 18, 321
- **SAFINA.** *J. Med. Chem.,* 2021, vol. 64, 2953
- **LUO.** *J. Med. Chem.,* 2019, vol. 62, 831
- **BANKAR.** *Cell Reports,* 2018, vol. 24, 3133
- **BROWN.** *Bioorg. Med. Chem.,* 2019, vol. 27 (1), 230
- **PAYNE.** *Br. J. Pharmacol.,* 2015, vol. 172 (10), 2654
- **BAGAL.** *Med. Chem. Lett.,* 2015, vol. 6 (6), 650
- **KORT.** *J. Med. Chem.,* 2008, vol. 51, 407
- **ZHANG.** *Neuropharmacology,* 2010, vol. 59, 201, , 207
- **KORNECOOK.** *J. Pharmacol. Exp. Ther.,* 2017, vol. 362, 146
- **DEUIS.** *Neuropharmacology,* 2017, vol. 127, 87, , 108
- **MCKERRALL.** *Bioorganic & Medicinal Chemistry Lett.,* 2018, vol. 28, 3141
- **BAGAL.** *Bioorganic & Medicinal Chemistry Lett.,* 2014, vol. 24, 3690